# EUROPEAN PATENT SPECIFICATION

(11) **EP 3 908 680 B1**
(45) Date of publication and mention of the grant of the patent: **01.03.2023**
(21) Application number: 20700162.9
(22) Date of filing: 10.01.2020
(51) Int. Cl.: C12Q 1/70, C12N 15/10, A61P 31/14, G01N 33/569

(54) **METHODS FOR SCREENING INHIBITORS AGAINST CHIKUNGUNYA VIRUS AND FOR DETERMINING WHETHER SUBJECTS ARE PREDISPOSED TO INFECTION BY SAID VIRUS**
VERFAHREN ZUM SCREENING VON INHIBITOREN GEGEN DAS CHIKUNGUNYA-VIRUS UND ZUM BESTIMMEN, OB PROBANDEN FÜR EINE INFEKTION MIT DIESEM VIRUS PRÄDISPONIERT SIND
PROCÉDÉS POUR DÉPISTER DES INHIBITEURS CONTRE LE VIRUS DU CHIKUNGUNYA ET POUR DÉTERMINER SI DES SUJETS SONT PRÉDISPOSÉS À UNE INFECTION PAR LEDIT VIRUS

(30) Priority: 11.01.2019 EP 19305036; 24.07.2019 EP 19305978
(43) Date of publication of application: 17.11.2021
(73) Proprietor: Institut National de la Santé et de la Recherche Médicale (INSERM), 75013 Paris (FR); CENTRE NATIONAL DE LA RECHERCHE SCIENTIFIQUE (CNRS), 75016 Paris (FR); Université Paris Cité, 75006 Paris (FR)
(72) Inventor: AMARA, Ali, 75010 PARIS (FR); MEERTENS, Laurent, 75010 PARIS (FR); HAFIRASSOU, Mohamed Lamine, 75010 PARIS (FR)
(74) Representative: Inserm Transfert
(86) International application number: PCT/EP2020/050541
(87) International publication number: WO 2020/144324

(56) References cited:
- RATHORE ABHAY P S ET AL: "Chikungunya virus nsP3 & nsP4 interacts with HSP-90 to promote virus replication: HSP-90 inhibitors reduce CHIKV infection and inflammationin vivo", ANTIVIRAL RESEARCH, vol. 103, 1 August 2018 (2018-08-01), - 13 June 2018 (2018-06-13), pages 7-16, XP028615960, ISSN: 0166-3542, DOI: 10.1016/J.ANTIVIRAL.2013.12.010
- CHETAN D. MESHRAM ET AL: "ABSTRACT", JOURNAL OF VIROLOGY., vol. 92, no. 16, 13 June 2018 (2018-06-13) , XP055581299, US ISSN: 0022-538X, DOI: 10.1128/JVI.00838-18
- A. MCCABE ET AL: "The Four and a Half LIM Family Members Are Novel Interactants of the Human T-Cell Leukemia Virus Type 1 Tax Oncoprotein", JOURNAL OF VIROLOGY., vol. 87, no. 13, 24 April 2013 (2013-04-24), pages 7435-7444, XP055581297, US ISSN: 0022-538X, DOI: 10.1128/JVI.00070-13
- MEERTENS LAURENT ET AL: "FHL1 is a major host factor for chikungunya virus infection", NATURE, MACMILLAN JOURNALS LTD, LONDON, vol. 574, no. 7777, 25 September 2019 (2019-09-25), pages 259-263, XP036902293, ISSN: 0028-0836, DOI: 10.1038/S41586-019-1578-4 [retrieved on 2019-09-25]

## Description

### FIELD OF THE INVENTION:

The present invention relates to methods for screening inhibitors against chikungunya virus as well as methods of diagnostics.

### BACKGROUND OF THE INVENTION:

Arthropod-borne viruses are the causative agents of some of the most important emerging infectious diseases and cause significant global public health problems. Of these viruses, the alphavirus genus belongs to the *Togaviridae* family, and the species in this genus cause diseases ranging from fever to severe polyarthritis to encephalitis. One alphavirus, chikungunya virus (CHIKV), caused recent outbreaks associated with severe morbidity. CHIKV is an enveloped, positive sense, single-stranded RNA virus with a genome of approximately 12 kb nucleotides long. The genome of CHIKV is organized as follows: 5' cap-nsPl-nsP2-nsP3-nsP4-(junction region)-C-E3-E2-6k-El-poly(A)-3 ', in which the first four proteins (nsPl-4) are nonstructural proteins, and the structural proteins are the capsid (C) and the envelope proteins (E). After infection with chikungunya virus, there is an incubation period lasting 2-4 days on average, followed by disease symptoms such as high fever, rash, headache, back pain, myalgia, and arthralgia. Severe clinical manifestations of chikungunya infection can also occur, for example, haemorrhagic fever, conjunctivitis, photophobia, hepatitis, stomatitis. Neurologic manifestations such as encephalitis, febrile seizures, meningeal syndrome and acute encephalopathy were also reported. Currently no vaccine or treatment exists to protect humans from CHIKV infection. Treatment is therefore purely symptomatic and is based on non-steroidal anti-inflammatory drugs. Accordingly, there is a high medical need exists to have new methods of screening of compounds which could inhibit chikungunya virus.

### SUMMARY OF THE INVENTION:

As defined by the claims, the present invention relates to methods for screening inhibitors against chikungunya virus as well as methods for determining whether a subject is predisposed to a CHIKV infection.

### DETAILED DESCRIPTION OF THE INVENTION:

Chikungunya virus (CHIKV) is a re-emerging Old World alphavirus transmitted to humans by mosquito bites which causes musculoskeletal and joint pain¹⁻³. Despite intensive investigations, the identity of the human cellular factors critical for CHIKV infection remains elusive, hampering both the understanding of viral pathogenesis and the development of anti-CHIKV therapies. Here, the inventors identified the Four-and-a-Half LIM domain protein 1 (FHL1)⁴ as a host factor required for CHIKV permissiveness and pathogenesis. Ablation of FHL1 expression results in massive inhibition of infection by several CHIKV strains and O'nyong-nyong virus, but not by other alphaviruses or flaviviruses. Conversely, expression of FHL1 enhances infection of cells that do not express it and are poorly susceptible to CHIKV. The inventors show that FHL1 directly interacts with the hypervariable domain of CHIKV nsP3 protein and is essential for viral RNA replication. FHL1 is highly expressed in CHIKV target cells and particularly abundant in muscles^{4,5}. Significantly, dermal fibroblasts and muscle cells derived from Emery-Dreifuss muscular dystrophy (EDMD) patients which lack functional FHL1⁶ are resistant to CHIKV infection. Importantly, CHIKV infection is undetectable in mice knocked out for the FHL1 gene. Overall, this study shows that FHL1 is a key host dependency factor for CHIKV infection and identifies nsP3-FHL1 interaction as a promising target for the development of anti-CHIKV therapies.

### Screening methods:

The first object of the present invention relates to a method for identifying a substance useful for inhibiting the replication capacity of chikungunya virus (CHIKV) comprising the steps of (a) contacting a polypeptide (P1) containing an amino acid sequence of the human FHL1 protein with a polypeptide (P2) having an amino acid sequence of the CHIKV NSP3 protein, under conditions and for a time sufficient to permit binding and the formation of a complex between the two polypeptides (P1) and (P2), in the presence of a test substance, and (b) detecting the formation of the complex, in which the ability of the test substance to inhibit the interaction between the two polypeptides (P1) and (P2) is indicated by a decrease in complex formation as compared to the amount of complex formed in the absence of the test substance and (c) selecting the substance that inhibits the interaction.

As used herein, the term "FHL1" has its general mean in the art and refers to the Four and a half LIM domains protein 1. The protein is also known as FCMSU, FHL-1, FHL1A, FHL1B, FLH1A, KYOT, RBMX1A, RBMX1B, SLIM, SLIM-1, SLIM1, SLIMMER, XMPMA. Mutations in FHL1 have been found in patients with Emery-Dreifuss muscular dystrophy. Multiple alternately spliced transcript variants which encode different protein isoforms have been described. In particular, FHL1A, FHL1B and FHL1C isoforms are represented by SEQ ID NO:1-3 respectively.
**SEQ ID NO:1>FHL1A** 
AEKFDCHYCRDPLQGKKYVQKDGHHCCLKCFDKFCANTCVECRKPIGADSKEVHYKNRFWHDTCFRCAK
CLHPLANETFVAKDNKILCNKCTTREDSPKCKGCFKAIVAGDQNVEYKGTVWHKDCFTCSNCKQVIGTGSFFPKG
EDFYCVTCHETKFAKHCVKCNKAITSGGITYQDQPWHADCFVCVTCSKKLAGQRFTAVEDQYYCVDCYKNFVAKK
CAGCKNPITGFGKGSSVVAYEGQSWHDYCFHCKKCSVNLANKRFVFHQEQVYCPDCAKKL
**SEQ ID NO:2>FHL1B** 
MAEKFDCHYCRDPLQGKKYVQKDGHHCCLKCFDKFCANTCVECRKPIGADSKEVHYKNRFWHDTCFRCA
KCLHPLANETFVAKDNKILCNKCTTREDSPKCKGCFKAIVAGDQNVEYKGTVWHKDCFTCSNCKQVIGTGSFFPK
GEDFYCVTCHETKFAKHCVKCNKAITSGGITYQDQPWHADCFVCVTCSKKLAGQRFTAVEDQYYCVDCYKNFVAK
KCAGCKNPITGKRTVSRVSHPVSKARKPPVCHGKRLPLTLFPSANLRGRHPGGERTCPSWVVVLYRKNRSLAAPR
GPGLVKAPVWWPMKDNPGTTTASTAKNAP
**SEQ ID NO:3 >FHL1C**

MAEKFDCHYCRDPLQGKKYVQKDGHHCCLKCFDKFCANTCVECRKPIGADSKEVHYKNRFWHDTCFRCA
KCLHPLANETFVAKDNKILCNKCTTREDSPKCKGCFKAIVAGDQNVEYKGTVWHKDCFTCSNCKQVIGTGSFFPK
GEDFYCVTCHETKFAKHCVKCNKGLVKAPVWWPMKDNPGTTTASTAKNAP

In some embodiments, the polypeptide (P1) comprises an amino acid sequences having at least 90% of identity with the amino acid sequence as set forth in SEQ ID NO:1, SEQ ID NO:2 or SEQ ID NO:3.

According to the invention a first amino acid sequence having at least 90% of identity with a second amino acid sequence means that the first sequence has 90; 91; 92; 93; 94; 95; 96; 97; 98; 99 or 100% of identity with the second amino acid sequence. Sequence identity is frequently measured in terms of percentage identity (or similarity or homology); the higher the percentage, the more similar are the two sequences. Methods of alignment of sequences for comparison are well known in the art. Various programs and alignment algorithms are described in: Smith and Waterman, Adv. Appl. Math., 2:482, 1981; Needleman and Wunsch, J. Mol. Biol., 48:443, 1970; Pearson and Lipman, Proc. Natl. Acad. Sci. U.S.A., 85:2444, 1988; Higgins and Sharp, Gene, 73:237-244, 1988; Higgins and Sharp, CABIOS, 5:151-153, 1989; Corpet et al. Nuc. Acids Res., 16:10881-10890, 1988; Huang et al., Comp. Appls Biosci., 8:155-165, 1992; and Pearson et al., Meth. Mol. Biol., 24:307-31, 1994). Altschul et al., Nat. Genet., 6:119-129, 1994, presents a detailed consideration of sequence alignment methods and homology calculations. By way of example, the alignment tools ALIGN (Myers and Miller, CABIOS 4:11-17, 1989) or LFASTA (Pearson and Lipman, 1988) may be used to perform sequence comparisons (Internet Program^{®} 1996, W. R. Pearson and the University of Virginia, fasta20u63 version 2.0u63, release date December 1996). ALIGN compares entire sequences against one another, while LFASTA compares regions of local similarity. These alignment tools and their respective tutorials are available on the Internet at the NCSA Website, for instance. Alternatively, for comparisons of amino acid sequences of greater than about 30 amino acids, the Blast 2 sequences function can be employed using the default BLOSUM62 matrix set to default parameters, (gap existence cost of 11, and a per residue gap cost of 1). When aligning short peptides (fewer than around 30 amino acids), the alignment should be performed using the Blast 2 sequences function, employing the PAM30 matrix set to default parameters (open gap 9, extension gap 1 penalties). The BLAST sequence comparison system is available, for instance, from the NCBI web site; see also Altschul et al., J. Mol. Biol., 215:403-410, 1990; Gish. & States, Nature Genet., 3:266-272, 1993; Madden et al. Meth. Enzymol., 266:131-141, 1996; Altschul et al., Nucleic Acids Res., 25:3389-3402, 1997; and Zhang & Madden, Genome Res., 7:649-656, 1997.

As used herein, the term "NSP3" has its general meaning in the art and refers to the non-structural protein 3 of chikungunya virus. In particular, the NSP3 is characterized by the sequence as set forth in SEQ ID NO:4. The hypervariable domain of NSP3 corresponds to the amino acid sequence ranging from the amino acid residue at position R326 to amino acid residue at position L524 in SEQ ID NO:4.
**> SEQ ID NO:4 NSP3 of CHIKV (the hypervariable region is underlined)**

MAPSYRVKRMDIAKNDEECVVNAANPRGLPGDGVCKAVYKKWPESFKNSATPVGTAKTVMCGTYPVIHA
VGPNFSNYSESEGDRELAAAYREVAKEVTRLGVNSVAIPLLSTGVYSGGKDRLTQSLNHLFTAMDSTDADVVIYC
RDKEWEKKISEAIQMRTQVELLDEHISIDCDIVRVHPDSSLAGRKGYSTTEGALYSYLEGTRFHQTAVDMAEIHT
MWPKQTEANEQVCLYALGESIESIRQKCPVDDADASSPPKTVPCLCRYAMTPERVTRLRMNHVTSIIVCSSFPLP
KYKIEGVQKVKCSKVMLFDHNVPSRVSPREYRSSQESAQEASTITSLTHSQFDLSVDGEILPVPSDLDADAPALE
PALDDGATHTLPSTTGNLAAVSDWVISTVPVAPPRRRRGRNLTVTCDEREGNITPMASVRFFRAELCPVVQETAE
TRDTAMSLQAPPSTATEPNHPPISFGASSETFPITFGDFNEGEIESLSSELLTFGDFLPGEVDDLTDSDWSTCSD
TDDEL

In some embodiments, the polypeptide (P2) comprises an amino acid sequence having at least 90% of identity with the amino acid sequence ranging from the amino acid residue at position R326 to amino acid residue at position L524 in SEQ ID NO:4.

In some embodiments, the polypeptide (P1) and/or (P2) is labelled with a detectable molecule.

According to the invention, said detectable molecule may consist of any substance or substance that is detectable by spectroscopic, photochemical, biochemical, immunochemical or chemical means. For example, useful detectable molecules include radioactive substance (including those comprising ³²P, ²⁵S, ³H, or ¹²⁵I), fluorescent dyes (including 5-bromodesosyrudin, fluorescein, acetyl amino fluorene or digoxigenin), fluorescent proteins (including GFPs and YFPs), or detectable proteins or peptides (including biotin, polyhistidine tails or other antigen tags like the HA antigen, the FLAG antigen, the c-myc antigen and the DNP antigen).

According to the invention, the detectable molecule is located at, or bound to, an amino acid residue located outside the binding sites of the polypeptides, in order to minimise or prevent any artefact for the binding between said polypeptides or between the test substance and or any of said polypeptides.

In some embodiments, the polypeptides of the invention are fused with a tag. Tag that are routinely used in the art can be used. For instance the polypeptide may be fused to a GST tag (Glutathione S-transferase) or polyhistidine tag. In said embodiments, the tag moiety of the said fusion protein may be used as detectable molecule. In the said fusion protein, the tag may be located either at the N-terminal end or at the C-terminal end. The tag detectable molecule may be detected when it is subsequently brought into contact with an anti-tag antibody, including with a labelled anti-tag antibody. Anti-tal antibodies labelled with various detectable molecules are easily commercially available.

In some embodiments, the polypeptides are fused with a portion of a transcription factor. The term "portion" when used herein for transcription factor, encompass complete proteins involved in multi protein transcription factors, as well as specific functional protein domains of a complete transcription factor protein. In some embodiments, the portion consists of either the DNA binding domain or the activator domain of a transcription factor. In some embodiments, the DNA binding domain and the activator domain both originate from the same naturally occurring transcription factor. In some embodiments, the DNA binding domain and the activator domain originate from distinct naturally occurring factors, while, when bound together, these two portions form an active transcription factor. Said protein moiety domain of transcription may be located either at the N-terminal end or at the C-terminal end. Such a DNA binding domain may consist of the well-known DNA binding domain of LexA protein originating form *E. Coli.* Moreover said activator domain of a transcription factor may consist of the activator domain of the well-known Gal4 protein originating from yeast.

As used herein, the expression "inhibiting the interaction" means that the substance reduces by at least about 10%, or by at least about 20%, or by at least about 30%, or by at least about 40%, or by at least about 50%, or by at least about 60%, or by at least about 70%, or by at least about 80%, or by at least about 90%, or by at least about 100% the interaction between the two polypeptides (P1) and (P2).

In some embodiments the step (b) consists in generating physical values which illustrate or not the ability of said test substance to inhibit the interaction between the polypeptides (P1) and (P2) and comparing said values with standard physical values obtained in the same assay performed in the absence of the said test substance. The "physical values" that are referred to above may be of various kinds depending of the binding assay that is performed, but notably encompass light absorbance values, radioactive signals and intensity value of fluorescence signal. If after the comparison of the physical values with the standard physical values, it is determined that the said test substance inhibits the binding between polypeptides (P1) and (P2), then the candidate is positively selected at step (c).

The substances that inhibit the interaction between the CHIKV protein and FHL1 protein encompass those substances that bind either to polypeptide (P1) or polypeptide (P2), provided that the binding of the said substances of interest then prevents the interaction between said polypeptides.

Different assays that are routinely used in the art can be used for detecting the formation of the complex formed by the polypeptides (P1) and (p2).

In some embodiments, a two-hybrid assay may be used wherein a first polypeptide is fused or conjugated to a first portion of a transcription factor (e.g. a DNA binding portion) and the second polypeptide is fused the second portion of the transcription factor (e.g. activator domain of a transcription factor), wherein the binding together of the first and second portions generates a functional transcription factor that binds to a specific regulatory DNA sequence, which in turn induces expression of a reporter DNA sequence, said expression being further detected and/or measured. A positive detection of the expression of said reporter DNA sequence means that an active transcription factor is formed, due to the binding together of said polypeptides.

Therefore in some embodiments of the invention, the assay of the invention comprises the following steps:
(1) providing a host cell expressing:
   - a first fusion polypeptide between (i) a first polypeptide (P1) or (P2) and (ii) a first protein portion of transcription factor
   - a second fusion polypeptide between (i) a second polypeptide (P1) or (P2) and (ii) a second portion of a transcription factor
      said transcription factor being active on DNA target regulatory sequence when the first and second protein portion are bound together and
      said host cell also containing a nucleic acid comprising (i) a regulatory DNA sequence that may be activated by said active transcription factor and (ii) a DNA report sequence that is operatively linked to said regulatory sequence
(2) bringing said host cell provided at step 1) into contact with a test substance to be tested
(3) determining the expression level of said DNA reporter sequence

The expression level of said DNA reporter sequence that is determined at step (3) above is compared with the expression of said DNA reporter sequence when step (2) is omitted. A reduced expression level of said DNA reporter sequence in the presence of the test substance means that the said test substance effectively inhibits the binding between CHIKV protein and FHL1 protein and that said test substance may be positively selected.

Suitable host cells include, without limitation, prokaryotic cells (such as bacteria) and eukaryotic cells (such as yeast cells, mammalian cells, insect cells, plant cells, etc.). However preferred host cell are yeast cells and more preferably a *Saccharomyces cerevisiae* cell or a *Schizosaccharomyces pombe* cell.

Similar systems of two-hybrid assays are well known in the art and therefore can be used to perform the assay according to the invention (see. Fields et al. 1989 ; Vasavada et al. 1991 ; Fearon et al. 1992; Dang et al., 1991, Chien et al. 1991, US 5,283,173, US 5,667,973, US 5,468,614, US 5,525,490 and US 5,637,463). For instance, as described in these documents, the Gal4 activator domain can be used for performing the assay according to the invention. Gal4 consists of two physically discrete modular domains, one acting as the DNA binding domain, the other one functioning as the transcription-activation domain. The yeast expression system described in the foregoing documents takes advantage of this property. The expression of a Gal1-LacZ reporter gene under the control of a Gal4-activated promoter depends on the reconstitution of Gal4 activity via protein-protein interaction. Colonies containing interacting polypeptides are detected with a chromogenic substrate for β-galactosidase. A compete kit (MATCHMAKER, TM) for identifying protein-protein interactions is commercially available from Clontech.

The expression of said detectable marker gene may be assessed by quantifying the amount of the corresponding specific mRNA produced. However, usually the detectable marker gene sequence encodes for detectable protein, so that the expression level of the said detectable marker gene is assessed by quantifying the amount of the corresponding protein produced. Techniques for quantifying the amount of mRNA or protein are well known in the art. For example, the detectable marker gene placed under the control of regulatory sequence may consist of the β-galactosidase as above described.

In some embodiments, the assay comprises a step of subjecting to a gel migration assay the mixture of the first polypeptide (P1) and the second polypeptide (P2) as above defined, with or without the test substance to be tested and then measuring the binding of the said polypeptides altogether by performing a detection of the complexes formed between said polypeptides. The gel migration assay can be carried out as known by the one skilled in the art.

Therefore in some embodiments of the invention, the assay of the invention comprises the following steps:
(1) providing the polypeptides (P1) and (P2) as defined above
(2) bringing into contact the test substance to be tested with said polypeptides
(3) performing a gel migration assay a suitable migration substrate with said polypeptides and said test substance as obtained at step (2)
(4) detecting and quantifying the complexes formed between said polypeptides on the migration assay as performed at step (3).

The presence or the amount of the complexes formed between the proteins are then compared with the results obtained when the assay is performed in the absence of the test substance to be tested. Therefore, when no complexes between the proteins is detected or, alternatively when those complexes are present in a lower amount compared to the amount obtained in the absence of the test substance, means that the test substance may be selected as an inhibitor of the specific interaction between said host protein and said viral protein.

The detection of the complexes formed between the said two proteins may be easily performed by staining the migration gel with a suitable dye and then determining the protein bands corresponding to the protein analysed since the complexes formed between the first and the second proteins possess a specific apparent molecular weight. Staining of proteins in gels may be done using the standard Coomassie brilliant blue (or PAGE blue), Amido Black, or silver stain reagents of different kinds. Suitable gels are well known in the art such as sodium dodecyl (lauryl) sulfate-polyacrylamide gel. In a general manner, western blotting assays are well known in the art and have been widely described (Rybicki et al., 1982; Towbin et al. 1979; Kurien et al. 2006).

In some embodiments, the protein bands corresponding to the proteins submitted to the gel migration assay can be detected by specific antibodies. It may use both antibodies directed against polypeptide (P1) and antibodies specifically directed against polypeptide (P2).

In some embodiments, both polypeptides are labelled with a detectable antigen as above described. Therefore, the proteins bands can be detected by specific antibodies directed against said detectable antigen. Preferably, the detectable antigen conjugates to the polypeptide (P1) is different from the antigen conjugated to the polypeptide (P2). For instance, the first polypeptide (P1) can be fused to a GST detectable antigen and the second polypeptide (P2) can be fused with the HA antigen. Then the protein complexes formed between the two proteins may be quantified and determined with antibodies directed against the GST and HA antigens respectively.

In some embodiments, the assay includes the use of an optical biosensor such as described by Edwards et al. (1997) or also by Szabo et al. (1995). This technique allows the detection of interactions between molecules in real time, without the need of labelled molecules. This technique is indeed bases on the surface plasmon resonance (SPR) phenomenon. Briefly, a first protein partner is attached to a surface (such as a carboxymethyl dextran matrix). Then the second protein partner is incubated with the previously immobilised first partner, in the presence or absence of the test substance to be tested. Then the binding including the binding level or the absence of binding between said protein partners is detected. For this purpose, a light beam is directed towards the side of the surface area of the substrate that does not contain the sample to be tested and is reflected by said surface. The SPR phenomenon causes a decrease in the intensity of the reflected light with a combination of angle and wavelength. The binding of the first and second protein partner causes a change in the refraction index on the substrate surface, which change is detected as a change in the SPR signal.

In some embodiments, the assay includes the use of affinity chromatography. Test substances for use in the assay above can also be selected by any immunoaffinity chromatography technique using any chromatographic substrate onto which the polypeptide (P1) and/or (P2) as above defined, has previously been immobilised, according to techniques well known from the one skilled in the art. Briefly, the polypeptide may be attached to a column using conventional techniques including chemical coupling to a suitable column matrix such as agarose, Affi Gel^{®}, or other matrices familiar to those of skill in the art. In some embodiments, the affinity column contains chimeric polypeptides in which the polypeptide (P1) or (P2) is fused to a tag such as glutathion-s-transferase (GST). Then a test substance is brought into contact with the chromatographic substrate of the affinity column previously, simultaneously or subsequently to the other protein among the said first and second protein. The after washing, the chromatography substrate is eluted and the collected elution liquid is analysed by detection and/or quantification of the said later applied first or second protein, so as to determine if, and/or to which extent, the test substance has impaired or not the binding between both polypeptides (P1) and (P2).

In some embodiments, the assay involves detection of a fluorescence signal. In some embodiments, the first polypeptide (P1) and the second polypeptide (P2) as above defined are labelled with a fluorescent molecule or substrate. Therefore, the potential alteration effect of the test substance to be tested on the binding between the first polypeptide (P1) and the second polypeptide (P2) as above defined is determined by fluorescence quantification.

For example, the first polypeptide (P1) and the second polypeptide (P2) as above defined may be fused with auto-fluorescent polypeptides, as GFP or YFPs as above described. The first polypeptide (P1) and the second polypeptide (P2) as above defined may also be labelled with fluorescent molecules that are suitable for performing fluorescence detection and/or quantification for the binding between said proteins using fluorescence energy transfer (FRET) assay. The first polypeptide (P1) and the second polypeptide (P2) as above defined may be directly labelled with fluorescent molecules, by covalent chemical linkage with the fluorescent molecule as GFP or YFP. The first polypeptide (P1) and the second polypeptide (P2) as above defined may also be indirectly labelled with fluorescent molecules, for example, by non covalent linkage between said polypeptides and said fluorescent molecule. A suitable receptor/ligand couple may be the biotin/streptavifin paired member or may be selected among an antigen/antibody paired member. For example, a protein according to the invention may be fused to a poly-histidine tail and the fluorescent molecule may be fused with an antibody directed against the poly-histidine tail.

In some embodiments, a first polypeptide is labelled with a first fluorophore substance and the second polypeptide is labelled with a second fluorophore substance. The first fluorophore substance may have a wavelength value that is substantially equal to the excitation wavelength value of the second fluorophore, whereby the bind of said first and second proteins is detected by measuring the fluorescence signal intensity emitted at the emission wavelength of the second fluorophore substance. Alternatively, the second fluorophore substance may also have an emission wavelength value of the first fluorophore, whereby the binding of said and second proteins is detected by measuring the fluorescence signal intensity emitted at the wavelength of the first fluorophore substance.

The fluorophores used may be of various suitable kinds, such as the well-known lanthanide chelates. These chelates have been described as having chemical stability, long-lived fluorescence (greater than 0.1 ms lifetime) after bioconjugation and significant energy-transfer in specificity bioaffinity assay. Document US 5,162,508 discloses bipyridine cryptates. Polycarboxylate chelators with TEKES type photosensitizers (EP0203047A1) and terpyridine type photosensitizers (EP0649020A1) are known. Document WO96/00901 discloses diethylenetriaminepentaacetic acid (DPTA) chelates which used carbostyril as sensitizer. Additional DPT chelates with other sensitizer and other tracer metal are known for diagnostic or imaging uses (e.g., EP0450742A1).

In some embodiments, the fluorescence assay consists of a Homogeneous Time Resolved Fluorescence (HTRF) assay, such as described in document WO 00/01663 or US 6,740,756. HTRF is a TR-FRET based technology that uses the principles of both TRF (time-resolved fluorescence) and FRET. More specifically, the one skilled in the art may use a HTRF assay based on the time-resolved amplified cryptate emission (TRACE) technology as described in Leblanc et al. (2002). The HTRF donor fluorophore is Europium Cryptate, which has the long-lived emissions of lanthanides coupled with the stability of cryptate encapsulation. XL665, a modified allophycocyanin purified from red algae, is the HTRF primary acceptor fluorophore. When these two fluorophores are brought together by a biomolecular interaction, a portion of the energy captured by the Cryptate during excitation is released through fluorescence emission at 620nm, while the remaining energy is transferred to XL665. This energy is then released by XL665 as specific fluorescence at 665 nm. Light at 665nm is emitted only through FRET with Europium. Because Europium Cryptate is always present in the assay, light at 620nm is detected even when the biomolecular interaction does not bring XL665 within close proximity.

The test substance of the invention may be selected from a library of substances previously synthesised, or a library of substances for which the structure is determined in a database, or from a library of substances that have been synthesised de novo. The test substance may be selected from the group of (a) proteins or peptides, (b) nucleic acids and (c) organic or chemical substances.

In some embodiments, the method of the present invention further comprises the step (d) consisting in determining whether the substance selected at step (c) inhibits the replication of CHIKV in a host cell and a step (e) that consists in positively selecting the test substance capable of inhibiting the replication of said CHIKV in said host cell.

In some embodiments, the method comprises the steps consisting of i) infecting said host cell with said CHIKV and ii) culturing said infected cell in presence of the test substance, iii) comparing the replicating capacity of the virus with the replication capacity determined in the absence of the test substance and iv) positively selecting the test substance that provides a decrease in the replication capacity of the virus.

The term "inhibiting the replication capacity," as used herein with reference to a viral phenotype, means that the virus grows to a lower titer in the presence of a substance as above described relative to the virus grown in the absence of said substance. In some embodiments, the presence of said substance which will inhibit the ability of an CHIKV to replicate in a host cell by at least about 10%, or by at least about 20%, or by at least about 30%, or by at least about 40%, or by at least about 50%, or by at least about 60%, or by at least about 70%, or by at least about 80%, or by at least about 90%, or by at least about 100%, or by at least about 200%, or by at least about 300%, or by at least about 400%, or by at least about 500% when compared to said CHIKV grown in the absence of said substance. Said replication capacity may be typically determined by any routine technique well known in the art.

According to the present invention, any CHIKV strain can be used. Preferably, said CHIKV strain corresponds to a clinical isolate of at least one circulating strain of CHIKV.

According to the invention, any eukaryotic cell may be used in the screening method of the invention. In some embodiments the cell is a human cell. In some embodiments, the cell is a cell line. Non-limiting examples of cell lines that can be suitable for the invention include but are not limited to BS-C-1, CV-1, Vero, Vero 76, Vero C1008, Vero 76, Cos-1, Cos-7, Huh7, FR11K-4, LLC-MK2 original, LLC-MK2 derivative, MDCK, RD, A549, MRC-5, KB, PER.C6, HEK-293 and CaCo-2 cells. Typically, cells are cultured in a standard commercial culture medium, such as Dulbecco's modified Eagle's medium supplemented with serum (e.g., 10% fetal bovine serum), or in serum free medium, under controlled humidity and C02 concentration suitable for maintaining neutral buffered pH (e.g., at pH between 7.0 and 7.2). Suitable serum free media are described, for example, in U.S. Provisional Application No. 60/638,166, filed Dec. 23, 2004, and in U.S. Provisional Application No. 60/641,139, filed Jan. 5, 2005. Optionally, the medium contains antibiotics to prevent bacterial growth, e.g., penicillin, streptomycin, etc., and/or additional nutrients, such as L-glutamine, sodium pyruvate, nonessential amino acids, additional supplements to promote favorable growth characteristics, e.g., trypsin, (3-mercaptoethanol, and the like.

In some embodiments, the infection of the cells with CHIKV is carried out at an m.o.i. (multiplicity of infection) of about 0.0001 to 10, preferably of 0.002 to 0.5. Typically, the MOI is 0.1, 0.01 or 0.001 for Vero cells or 0.1, 0.05, 0.01 or 0.001 for Huh7 cells while preferably an MOI is used of 0.001 for Vero cells or 0.05 for Huh7 cells.

Typically, the cells can be grown in culture under conditions permissive for replication and assembly of viruses. In some embodiments, cells can be cultured at a temperature below about 37° C, preferably at a temperature equal to, or less than, about 35° C. Typically, the cells are cultured at a temperature between about 32° C. and about 35° C. In some embodiments, the cells are cultured at a temperature between about 32° C. and 34° C, e.g., at about 33° C.

As described above, the methods of the present invention are particularly useful for screening a plurality of substances that may be used for the treatment or prevention of CHIKV infections as described infra.

In some embodiments, the substances selected by the above mentioned screening method may be used in the treatment of CHIKV infection. For example, therapeutic treatments includes the reduction or amelioration of the progression, severity and/or duration of CHIKV infections, or the amelioration of one or more symptoms (specifically, one or more discernible symptoms) of CHIKV infections, resulting from the administration of at least one substance selected by the above mentioned screening method. In some embodiments, the therapeutic treatment includes the amelioration of at least one measurable physical parameter of a CHIKV infection. In some embodiments, the therapeutic treatment includes the inhibition of the progression of an CHIKV infection, either physically by, e.g., stabilization of a discernible symptom, physiologically by, e.g., stabilization of a physical parameter, or both. In some embodiments, the therapeutic treatment includes the reduction or stabilization of CHIKV infections.

In some embodiments, the substances selected by the above mentioned screening method may be used in a prophylactic treatment. The terms "prophylaxis" or "prophylactic use" and "prophylactic treatment" as used herein, refer to any medical or public health procedure whose purpose is to prevent, rather than treat or cure a disease. As used herein, the terms "prevent", "prevention" and "preventing" refer to the reduction in the risk of acquiring or developing a given condition, or the reduction or inhibition of the recurrence or said condition in a subject who is not ill, but who has been or may be near a subject with the disease. As used herein, prophylactic use includes the use in situations in which an outbreak has been detected, to prevent contagion or spread of the infection in places where a lot of people that are at high risk of serious complications live in close contact with each other (e.g. in a hospital ward, daycare center, prison, nursing home, etc). Prophylactic use may also include treating a person who is not ill with the CHIKV or not considered at high risk for complications, in order to reduce the chances of getting infected with the CHIKV and passing it on to a high-risk person in close contact with him (for instance, healthcare workers, nursing home workers, etc).

Typically, the substances selected by the above mentioned screening method are administered to the subject in an effective amount. As used herein, an "effective amount" refers to an amount sufficient to elicit the desired biological response. In the present invention the desired biological response is to inhibit the replication of CHIKV, to reduce the amount of CHIKV or to reduce or ameliorate the severity, duration, progression, or onset of a CHIKV infection, prevent the advancement of an CHIKV infection, prevent the recurrence, development, onset or progression of a symptom associated with an CHIKV infection, or enhance or improve the prophylactic or therapeutic effect(s) of another therapy used against CHIKV infections. The precise amount of compound administered to a subject will depend on the mode of administration, the type and severity of the infection and on the characteristics of the subject, such as general health, age, sex, body weight and tolerance to drugs. The skilled artisan will be able to determine appropriate dosages depending on these and other factors. When co-administered with other anti viral agents, e.g., when coadministered with an anti-CHIKV medication, an "effective amount" of the second agent will depend on the type of drug used. Suitable dosages are known for approved agents and can be adjusted by the skilled artisan according to the condition of the subject, the type of condition(s) being treated and the amount of a compound described herein being used. In cases where no amount is expressly noted, an effective amount should be assumed. For example, compounds described herein can be administered to a subject in a dosage range from between approximately 0.01 to 100 mg/kg body weight/day for therapeutic or prophylactic treatment.

In some embodiments the substances selected by the above mentioned screening method are used in combination with an additional suitable therapeutic agent, for example, an antiviral agent or a vaccine. When "combination therapy" is employed, an effective amount can be achieved using a first amount of a substance selected by the above mentioned screening method and a second amount of an additional suitable therapeutic agent (e.g. an antiviral agent). As used herein, the terms "in combination" or "co-administration" can be used interchangeably to refer to the use of more than one therapy (e.g., one or more prophylactic and/or therapeutic agents). The use of the terms does not restrict the order in which therapies (e.g., prophylactic and/or therapeutic agents) are administered to a subject. Specific examples that can be co-administered with a substance selected by the above mentioned screening method include non-steroidal anti-inflammatory drugs (NSAIDS). Examples of Aspirin, Naproxen, Sulindac, Ibuprofen, Indomethacin, Valproic acid, Fenamic acid, Flurbiprofen, Diclofenac, Diflunisal, Salsalate, Choline Magnesium Trisalicylate, Dexibuprofen, Fenoprofen, Detoprofen, Dexketoprofen, Oxaprozin, Loxoprofen, Tolmetin, Etodolac, Ketorolac, Aceclofenac, Nabumetone, Piroxicam, Meloxicam, Tenoxicam, Droxicam, Lornoxicam, Isoxicam, Mefenamic acid, Meclofenamic acid, Flufenamic acid, Tolfenamic acid, Selective COX-2 inhibitors, and Licofelone.

The substances selected by the above mentioned screening method can be formulated into pharmaceutical compositions that further comprise a pharmaceutically acceptable carrier, diluent, adjuvant or vehicle. In some embodiments, the present invention relates to a pharmaceutical composition comprising a substance selected by the above mentioned screening method described above, and a pharmaceutically acceptable carrier, diluent, adjuvant or vehicle. In some embodiments, the present invention is a pharmaceutical composition comprising an effective amount of a compound of the present invention or a pharmaceutically acceptable salt thereof and a pharmaceutically acceptable carrier, diluent, adjuvant or vehicle. Pharmaceutically acceptable carriers include, for example, pharmaceutical diluents, excipients or carriers suitably selected with respect to the intended form of administration, and consistent with conventional pharmaceutical practices.

### Diagnostic methods:

A further object of the present invention relates to a method of testing whether a subject is predisposed a CHIKV infection comprising the steps consisting of i) measuring the expression level of FHL1 in a sample obtained from the subject and ii) comparing the expression level measured at step i) with a predetermined reference value and iii) concluding that the subject is predisposed to a CHIKV infection when differential between the measured expression level and the predetermined reference value is detected.

The method of the present invention is thus particularly suitable for discriminating subjects having a high risk of having a CHIKV infection from subjects having a low risk of having a CHIKV infection. The method the present invention is thus particularly suitable for carrying prophylactic behaviours including prophylactic treatments and/or isolations during a CHIKV outbreak.

As used herein, the term "risk" relates to the probability that an event will occur over a specific time period, as in the conversion to a CHIKV infection, and can mean a subject's "absolute" risk or "relative" risk. Absolute risk can be measured with reference to either actual observation post-measurement for the relevant time cohort, or with reference to index values developed from statistically valid historical cohorts that have been followed for the relevant time period. Relative risk refers to the ratio of absolute risks of a subject compared either to the absolute risks of low risk cohorts or an average population risk, which can vary by how clinical risk factors are assessed. Odds ratios, the proportion of positive events to negative events for a given test result, are also commonly used (odds are according to the formula p/(l-p) where p is the probability of event and (1- p) is the probability of no event) to no- conversion. "Risk evaluation," or "evaluation of risk" in the context of the present invention encompasses making a prediction of the probability, odds, or likelihood that an event or disease state may occur, the rate of occurrence of the event or conversion from one disease state to another, i.e., from a normal condition to a CHIKV infection or to one at risk of developing a CHIKV infection. Risk evaluation can also comprise prediction of future clinical parameters, traditional laboratory risk factor values, or other indices of a CHIKV infection, either in absolute or relative terms in reference to a previously measured population. The methods of the present invention may be used to make continuous or categorical measurements of the risk of conversion to a CHIKV infection, thus diagnosing and defining the risk spectrum of a category of subjects defined as being at risk for a CHIKV infection. In the categorical scenario, the invention can be used to discriminate between normal and other subject cohorts at higher risk for a CHIKV infection. In some embodiments, the present invention may be used so as to discriminate those at risk for developing a CHIKV infection from normal.

The expression level may be measured by routine technique well known in the art.

For instance, assays for measuring the expression level comprises quantifying the protein and thus typically invlove use of standard immunodiagnostic techniques, including immunoassays such as competition, direct reaction, or sandwich type assays. Such assays include, but are not limited to, agglutination tests; enzyme-labelled and mediated immunoassays, such as ELISAs; biotin/avidin type assays; radioimmunoassays; immunoelectrophoresis; immunoprecipitation. Use of a binding partner that is specific for HFHL1 is typically involved. The binding partners of the invention such as antibodies or aptamers, may be labelled with a detectable molecule or substance, such as a fluorescent molecule, a radioactive molecule or any others labels known in the art. Labels are known in the art that generally provide (either directly or indirectly) a signal. As used herein, the term "labelled", with regard to the antibody, is intended to encompass direct labelling of the antibody or aptamer by coupling (i.e., physically linking) a detectable substance, such as a radioactive agent or a fluorophore (e.g. fluorescein isothiocyanate (FITC) or phycoerythrin (PE) or Indocyanine (Cy5)) to the antibody or aptamer, as well as indirect labelling of the probe or antibody by reactivity with a detectable substance. An antibody or aptamer of the invention may be labelled with a radioactive molecule by any method known in the art. For example radioactive molecules include but are not limited radioactive atom for scintigraphic studies such as I123, I124, In111, Re186, Re188. The aforementioned assays generally involve the bounding of the binding partner (ie. Antibody or aptamer) in a solid support. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like.

In some embodiments, the assay consists in quantifying the amount of the mRNA. Methods for determining the quantity of mRNA are well known in the art. For example the nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The extracted mRNA is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous. Other methods of Amplification include ligase chain reaction (LCR), transcription-mediated amplification (TMA), strand displacement amplification (SDA) and nucleic acid sequence based amplification (NASBA).

In some embodiments, the predetermined reference value is a threshold value. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. For example, after determining the total iron content in a group of reference, one can use algorithmic analysis for the statistic treatment of the measured levels of the immune marker in samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is moderate. When AUC is higher than 0.9, the accuracy is quite high. This algorithmic method is preferably done with a computer. Existing software or systems in the art may be used for the drawing of the ROC curve, such as: MedCalc 9.2.0.1 medical statistical software, SPSS 9.0, ROCPOWER.SAS, DESIGNROC.FOR, MULTIREADER POWER.SAS, CREATE-ROC.SAS, GB STAT VI0.0 (Dynamic Microsystems, Inc. Silver Spring, Md., USA), etc.

In some embodiments, the higher the expression level of FHL1 is, the higher the risk of having a CHIKV infection is.

Accordingly, in a particular embodiment, the invention relates to a method of testing a subject thought to have or be predisposed to having a CHIKV infection, which comprises the step of analysing a sample of interest obtained from said subject for detecting the presence of a genetic variant in the gene encoding for FHL1 protein.

As used herein, the term "genetic variant" has its general meaning in the art and denotes any of two or more alternative forms of a gene occupying the same chromosomal locus. The alteration typically consists in a substitution, an insertion, and/or a deletion, at one or more (e.g., several) positions in the gene. Genetic variation arises naturally through mutation, and may result in phenotypic polymorphism within populations. Gene mutations can be silent (no change in the encoded polypeptide) or may encode polypeptides having altered amino acid sequence. The term is also known as "polymorphism".

In some embodiments, the genetic variant is located in the promoter.

In some embodiments, the genetic variant is located in an intron.

In some embodiments, the genetic variant is located in an exon.

In some embodiments, the genetic variant is present is heterozygous (i.e. present in only one allele) or homozygous (i.e. present in the 2 alleles).

In some embodiments, the method of the present invention comprises detecting one or more single nucleotide polymorphisms (SNP).

In some embodiments, the genetic variant is a single nucleotide polymorphism. As used herein, the term "single nucleotide polymorphism" or "SNP" has its general meaning in the art and refers to a single nucleotide variation in a genetic sequence that occurs at appreciable frequency in the population.

Detecting the genetic variant may be determined according to any genotyping method known in the art. Typically, common genotyping methods include, but are not limited to, TaqMan assays, molecular beacon assays, nucleic acid arrays, allele-specific primer extension, allele-specific PCR, arrayed primer extension, homogeneous primer extension assays, primer extension with detection by mass spectrometry, sequencing, multiplex primer extension sorted on genetic arrays, ligation with rolling circle amplification, homogeneous ligation, OLA, multiplex ligation reaction sorted on genetic arrays, restriction-fragment length polymorphism, single base extension-tag assays, and the Invader assay. Such methods may be used in combination with detection mechanisms such as, for example, luminescence or chemiluminescence detection, fluorescence detection, time-resolved fluorescence detection, fluorescence resonance energy transfer, fluorescence polarization, mass spectrometry, and electrical detection. Various methods for detecting polymorphisms include, but are not limited to, methods in which protection from cleavage agents is used to detect mismatched bases in RNA/RNA or RNA/DNA, comparison of the electrophoretic mobility of variant and wild type nucleic acid molecules, and assaying the movement of polymorphic or wild-type fragments in polyacrylamide gels containing a gradient of denaturant using denaturing gradient gel electrophoresis. Sequence variations at specific locations can also be assessed by nuclease protection assays such as RNase and SI protection or chemical cleavage methods. Detecting the genetic variant may also be performed by sequencing. A variety of automated sequencing procedures can be used, including sequencing by mass spectrometry. The nucleic acid sequences of the present invention enable one of ordinary skill in the art to readily design sequencing primers for such automated sequencing procedures. Commercial instrumentation, such as the Applied Biosystems 377, 3100, 3700, 3730, and 3730×1 DNA Analyzers (Foster City, Calif.), is commonly used in the art for automated sequencing. Nucleic acid sequences can also be determined by employing a high throughput mutation screening system, such as the SpectruMedix system.

A further object of the present invention relates to a method of testing a subject thought to have or be predisposed to having a CHIKV infection, which comprises the step of analysing a sample of interest obtained from said subject for detecting post-translational modifications of FHL1 protein.

The post-translational modifications FHL1 protein include but are not limited to phosphorylation, acetylation, glycosylation. and the like. Detecting the post-translational modifications of the FHL1 protein may be assessed by using a binding partner specific for a post-translational form of FHL1 protein. As described above, the binding partner may be an antibody (e.g., a radio-labeled, chromophore- labeled, fluorophore-labeled, or enzyme-labeled antibody), an antibody derivative (e.g., an antibody conjugate with a substrate or with the protein or ligand of a protein of a protein/ligand pair (e.g., biotin-streptavidin)), or an antibody fragment (e.g., a single-chain antibody, an isolated antibody hypervariable domain, etc.) which binds specifically to a specific form of the FHL1 protein. Said analysis can be assessed by a variety of techniques well known from one of skill in the art including, but not limited to, enzyme immunoassay (EIA), radioimmunoassay (RIA), Western blot analysis and enzyme linked immunoabsorbant assay (RIA).

The invention will be further illustrated by the following figures and examples. However, these examples and figures should not be interpreted in any way as limiting the scope of the present invention.

### FIGURES:

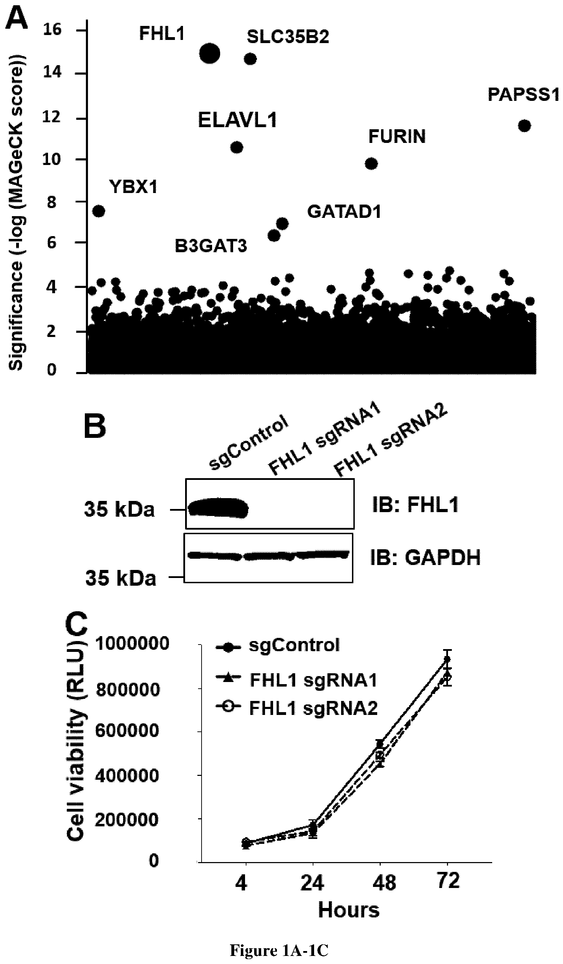

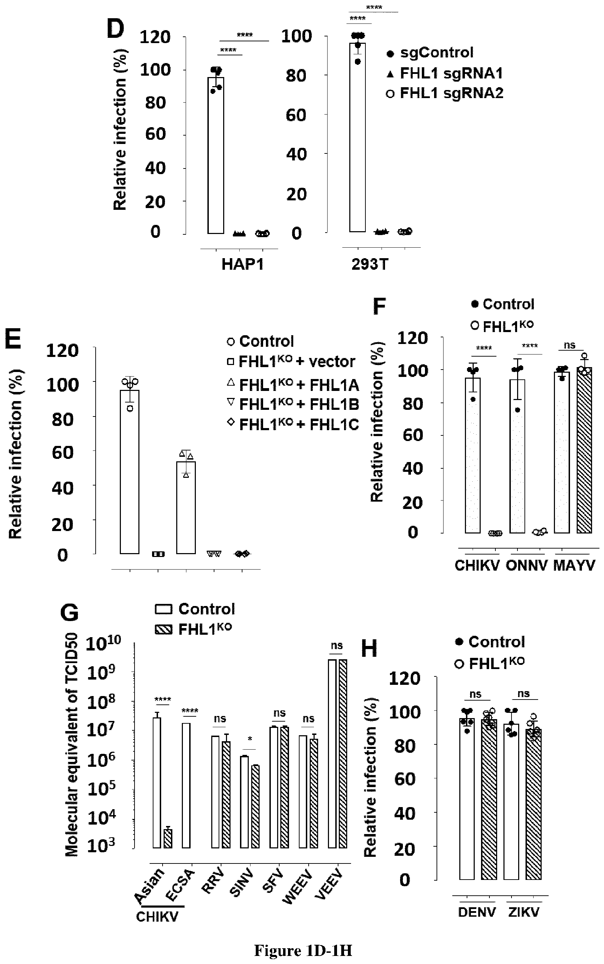

**Figure 1****. CRISPR-Cas9 genetic screen identifies FHL1 has an essential host factor for CHIKV and ONNV infection.**
   **(A)** Results of the CHIKV 21 strains screen analyzed by MAGeCK. Each circle represents individual gene. Y-axis represents the significance of sgRNA enrichment of genes in the selected population compared to the non-selected control population. X-axis represents a random distribution of the genes. (B-D) HAP1 cells were edited with a control or two different FHL1 sgRNA. **(B)** Immunoblotting of FHL1 in control and FHL1^{KO} clones. **(C)** Viability of control and FHL1^{KO} HAP1 cells over a 72 hours period using the Cell-Titer Glo assay. Data shown are representative of two experiments. **(D)** Control or FHL1^{KO} cells were exposed to CHIKV 21 strains (HAP1: MOI of 10; 293T: MOI of 2) and stained for E2 protein. Data shown are mean +/- SD from three experiments (n=6, one-way ANOVA with Dunnett's test). **(E)** Control, FHL1^{KO} or FHL1^{KO} HAP1 cells transduced with the three FHL1 isoforms were inoculated with CHIKV 21 strains (MOI of 10) and E2 expression was analyzed. Data shown are mean +/- SD from two experiments (n=4, one-way ANOVA with Dunnett's test). **(F)** Control or FHL1^{KO} cells were inoculated with CHIKV (MOI of 10), MAYV (MOI of 10) and ONNV (MOI of 10), and E2 expression was analyzed. Data shown are mean +/- SD from two experiments (n=4, one-way ANOVA with Dunnett's test). **(G)** Control or FHL1^{KO} HAP1 cells were inoculated with TCID50 of the indicated alphaviruses, and infection was assessed by qRT-PCR. Data shown are mean +/- SD from one representative experiment. **(H)** Control or FHL1^{KO} HAP1 cells were inoculated with DENV (MOI of 10) and ZIKV (MOI of 20), and E expression was analyzed. Data shown are mean +/- SD from three experiments (n=6, one-way ANOVA with Dunnett's test) * P<0.05; **** P< 0.0001; ns not significant.

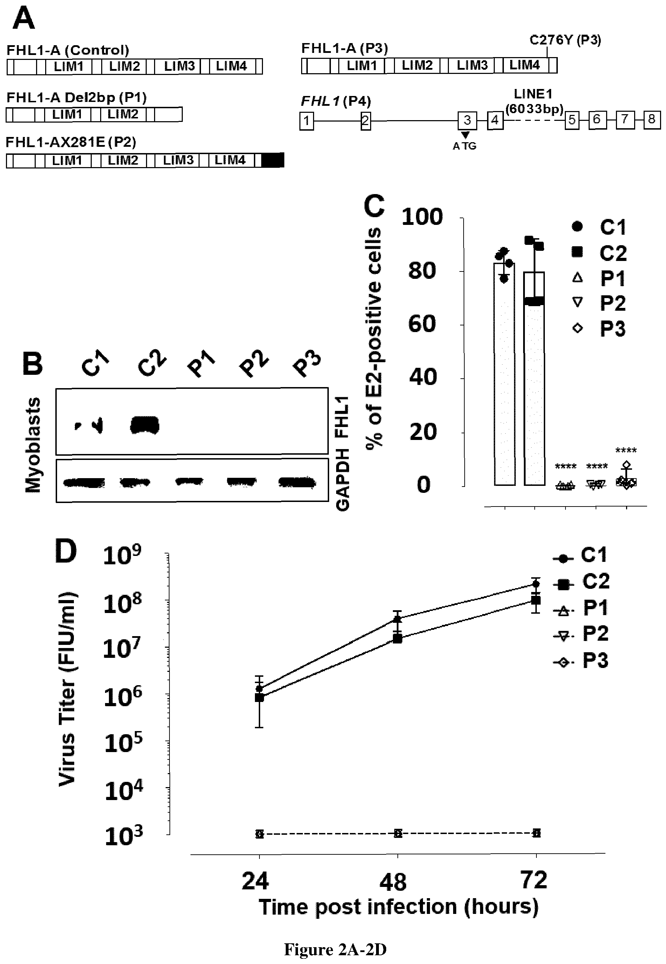

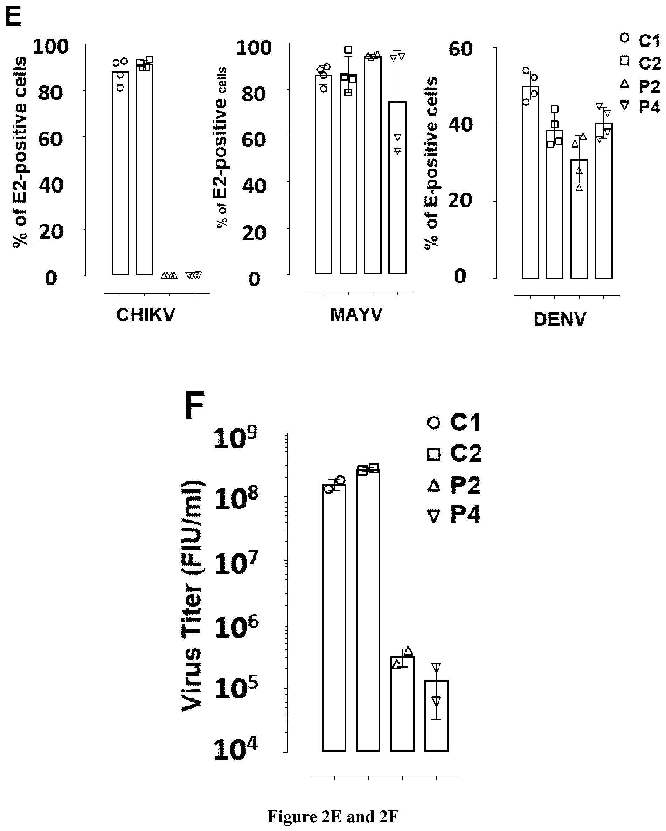

**Figure 2****. Primary myoblast and fibroblast from FHL1 deficient patient are resistant to CHIKV infection.**
   **(A)** Schematic representation of FHL1 protein in control (C1 and C2) or patient carrying a mutation (P1 to P3), and genomic organization of FHL1 gene carrying a LINE1 insertion in exon 4 (P4). **(B)** Immunoblotting of FHL1 in the lysate from control and patient primary myoblast. **(C)** Control and patient primary myoblast were inoculated with CHIKV (MOI of 2), and E2 expression was analyzed. Data shown are mean +/- SD from two experiments (n=4, one-way ANOVA with multi-comparison test). **(D)** Quantification of viral particles released by infected primary myoblast at 24, 48 and 72 hours post-infection. FIU, flow cytometry infectious particles. Data shown are mean +/- SD from one representative of two experiments (n=2). **(E)** Primary fibroblasts were inoculated with CHIKV (MOI of 0.4), MAYV (MOI of 2) and DENV (MOI of 20), and analyzed for E2 or E protein expression. Data shown are mean +/- SD from two experiments (n=4, one-way ANOVA with multi-comparison test). **(F)** Quantification of viral particles released by infected primary fibroblast at 48 hours post-infection. FIU, flow cytometry infectious particles. Data shown are mean +/- SD from one representative of two experiments (n=2). **** P< 0.0001; ns not significant.

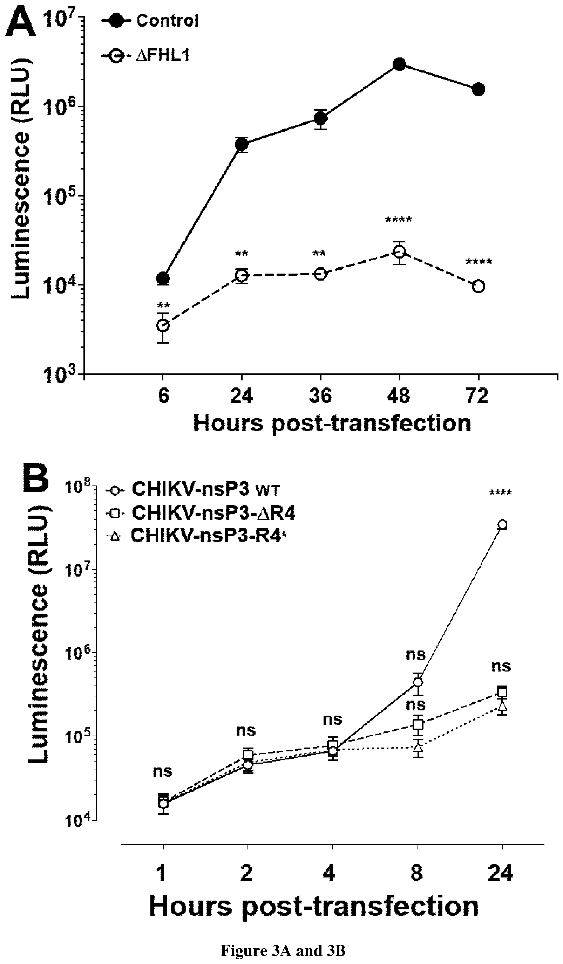

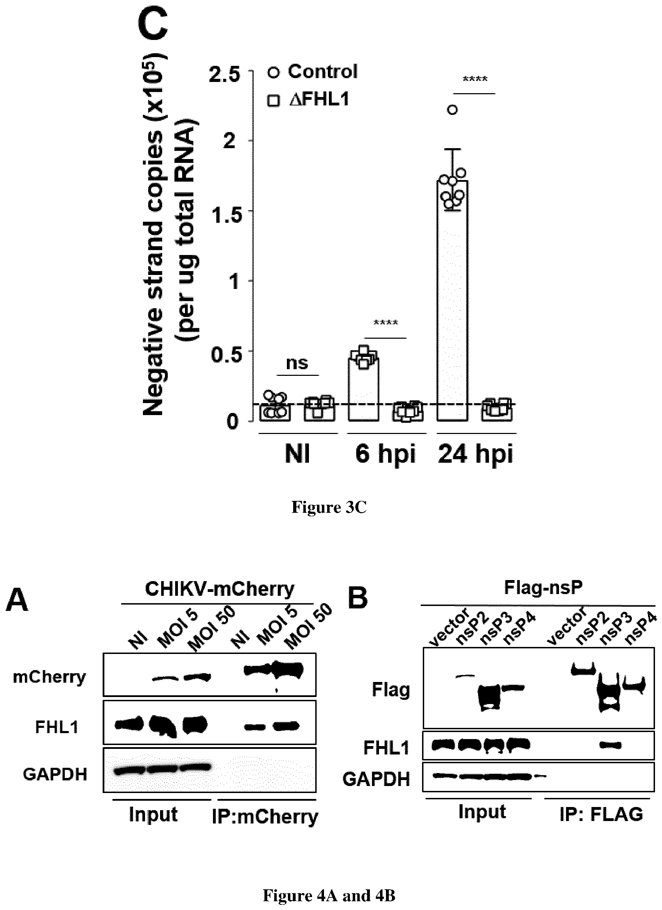

**Figure 3****. Depletion of FHL1 prevents CHIKV** replication.
   **(A)** Transfection of CHIKV replicon RNA expressing luciferase into control and FHL1^{KO} HAP1 cells. Luciferase activity was monitored at indicated time point. RLU, relative light units. Data shown are mean +/- SD from three experiments (n=12). **(B)** Control 293T cells were transfected with the indicated CHIKV capped in vitro transcribed RNA expressing renilla luciferase (Rluc). Rluc activity was monitored at indicated time points. RLU, relative light units. Data shown are mean +/- SEM (n=2 independent experiments in quadruplicate; Two-way ANOVA with Tukey's multiple comparisons test). **(C)** Negative-stranded viral RNA quantification by RT-qPCR from samples (h.p.i., hours post-infection; NI, not infected). Data are mean ± s.d. n =2 independent experiments in quadruplicate. One-way ANOVA with a Tukey's multiple comparison test. Dashed line represents the experimental background threshold.

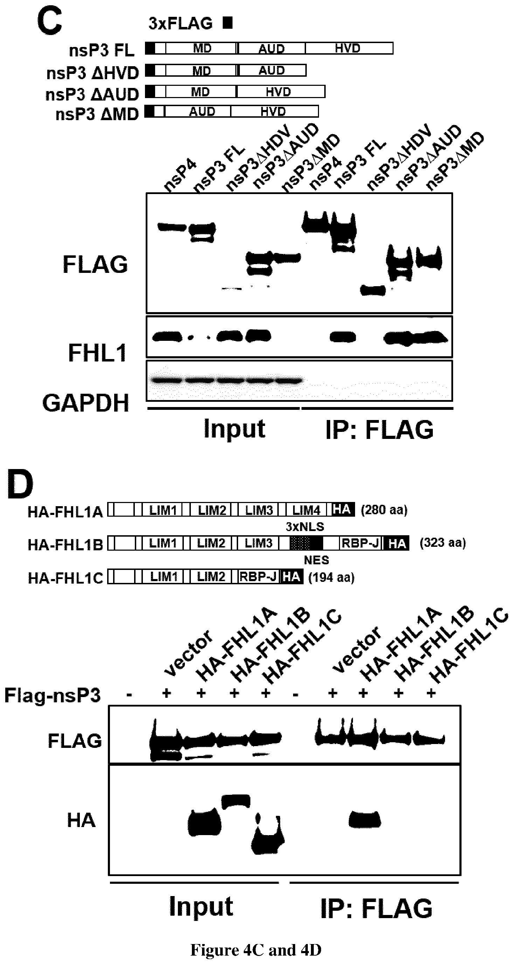

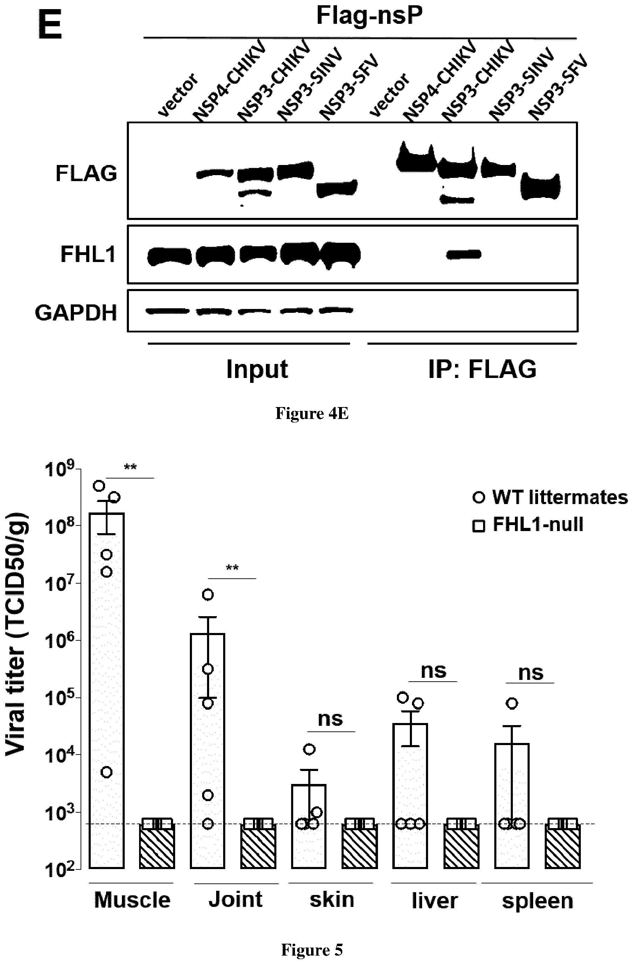

**Figure 4****. CHIKV non-structural protein 3 interacts specifically with FHL1A through its hypervariable domain.**
   (A) Immunoassay of the interaction between viral replication complex and endogenous FHL1 in 293T cells infected with CHIKV expressing nsp3-mCherry, assessed by immunoprecipitation with anti-RFP antibody followed by immunoblot analysis with anti-mCherry and anti-FHL1. **(B)** Immunoassay of the interaction of endogenous FHL1 with CHIKV nsP proteins in 293T cells transfected with plasmids encoding Flag-tagged individual nsP, assessed by co-immunoprocipitation with anti-FLAG and immunoblot analysis with anti-FHL1 and anti-FLAG. **(C)** Top panel shows full-length CHIKV nsP3 and constructs of nsP3 containing various combination of MD, AUD and HVD. Bottom panel shows immunoassay in 293T cells transfected with the plasmids encoding FLAG-tagged nsP3 constructs. Cellular lysates were subject to immunoprecipitation with anti-FLAG followed by immunoblot analysis with anti-FLAG and anti-FHL1. **(D)** Immunoassay of the interaction between CHIKV nsP3 and FHL1 isoform in 293Tcells transfected with FLAG-tagged nsP3 and empty vector or HA-tagged plasmids encoding the three FHL1 isoforms (top panel). Cellular lysates were subject to immunoprecipitation with anti-HA followed by immunoblot analysis with anti-FLAG and anti-HA. **(E)** Immunoassay of the interaction between FHL1 and nsP3 protein from various alphaviruses in 293T cells transfected with plasmid encoding FLAG-tagged CHIKV, Sindbis (SINV) and Semliki forest virus (SFV) nsP3. Cellular lysates were subject to immunoprecipitation with anti-FLAG followed by immunoblot analysis with anti-FLAG and anti-FHL1. (A-E) Data are representative of two experiments with similar results.
**Figure 5****. FHL1 is a factor of susceptibility to CHIKV infection in mice.** Viral titres in tissues of 9-day-old mice. Wild-type (WT) male littermates (n = 5) and Fhl1-/y mice (n = 7) were inoculated with 105 plaque-forming units of CHIKV by intradermal injection and euthanized 7 days after infection. The amount of infectious virus in tissues was quantified as the TCID50. The dashed line indicates the detection threshold. Data are mean ± s.e.m. Two-tailed t-test.

### EXAMPLE 1:

Chikungunya virus (CHIKV) has caused recent outbreaks associated with severe morbidity. Currently no vaccine or treatment exists to protect humans from CHIKV infection. Treatment is therefore purely symptomatic and is based on non-steroidal anti-inflammatory drugs. Accordingly, there is a high medical need exists to have new methods of screening of compounds which could inhibit chikungunya virus. Further to a CRISPR-Cas9 genetic screen the inventors now identify the four and a half LIM domains protein 1 (FHL1) has an essential host factor for CHIKV infection (**Figure 1A-H**). In particular, they show that primary myoblast and fibroblast from FHL1 deficient patient are resistant to CHIKV infection (**Figure 2A-F**). They also show that transfection of CHIKV(GAA) RNA in ΔFHL1 or control cells resulted in similar Rluc activities (Data not shown), indicating that FHL1 is dispensable for viral RNA translation. When similar experiments were performed with wild-type CHIKV RNA, a large increase in Rluc activity was observed in control-but not ΔFHL1-cells 24 h after infection, demonstrating that FHL1 is essential for viral RNA replication. Quantitative reverse-transcription PCR (RT-qPCR) experiments showed that ablation of FHL1 resulted in severely reduced synthesis of CHIKV negative-strand RNA.They demonstrate that depletion of FHL1 prevents CHIKV replication (**Figure 3A-C**).

Finally, they show that CHIKV non-structural protein 3 interacts specifically with FHL1A through its hypervariable domain (**Figure 4A-E**). Thus compounds that are capable of inhibiting the interaction between the non-structural protein 3 and FHL1 would be suitable for inhibiting the replication capacity of the virus. Determining the expression level of FHL1 and/or identifying some genetic variant would also be suitable for determining whether some subjects are predisposed to CHIKV infection.

### EXAMPLE 2:

### Methods:

**Cell culture.** HAP1 cells (Horizon Discovery), which are derived from near-haploid chronic myeloid leukemia KBM7 cells, were cultured in IMDM supplemented with 10% FBS, 1% penicillin-streptomycin (P/S) and GlutaMAX (Thermo Fisher Scientific). 293FT (Thermo Fisher Scientific), HEK-293T (ATCC), Vero E6 (ATCC), HepG2 (kind gift of Olivier Schwartz, Institut Pasteur, Paris, France), primary myoblasts and primary fibroblasts were cultured in DMEM supplemented with 10% FBS, 1% penicillin-streptomycin, 1% GlutaMAX and 25 mM Hepes. Human placenta choriocarcinoma Bewo cells were cultured in in DMEM supplemented with 5% FBS, 1% penicillin-streptomycin, 1% GlutaMAX and 25 mM Hepes. AP61 mosquito (Aedes pseudoscutellaris) cells (gift from Philippe Despres, Institut Pasteur, Paris, France) were cultured at 28°C in Leibovitz medium supplemented with 10% FCS, 1% P/S, 1% glutamine, 1X non-essential amino acid, 1X Tryptose phosphate and 10 mM Hepes. All cell lines were cultured at 37°C in presence of 5% CO₂ with the exception of AP61 that were maintained at 28°C with no CO₂.

**Virus strains and culture.** CHIKV21 (strain 06-21), ZIKV (HD78788) (both are kind gift from Philippe Despres, Institut Pasteur, Paris, France), CHIKV West Africa (strain 37997, accession nb AY726732.1) and dengue virus serotype 2 DENV (16681) viruses were propagated in mosquito AP61 cell monolayers with limited cell passages. CHIKV-Brazza-MRS1 2011, CHIKV-Ross, CHIKV-St Martin H20235 2013-Asian, RRV (strain 528v), MAYV (strain TC 625), ONNV (strain Dakar 234), SINV (strain Egypt 339), EEEV (strain H178/99), VEEV (strain TV83 vaccine), WEEV (strain 47A), SFV (strain 1745) were obtained from the European Virus Archive (EVA) collection and propagated with limited passage on Vero E6 cells.

pCHIKV-M-Gluc (see plasmid sections) and pCHIKV-mCherry molecular clones were derivate of pCHIKV-M constructed from a CHIKV (strain BNI-CHIKV_899) isolated from a patient during Mauritius outbreak in 2006. To generate infectious virus from CHIKV molecular clones, capped viral RNAs were generated from the NotI-linearized CHIKV plasmids using a mMESSAGE mMACHINE SP6 or T7 Transcription Kit (Thermo Fischer Scientific) according to manufacturer's instructions. Resulting RNAs were purified by phenol:chloroform extraction and isopropanol precipitation, resuspended in water, aliquoted and stored at -80°C until use. Thirty µg of purified RNAs were transfected in BHK21 with lipofectamine 3000 reagent and supernatants harvested 72 hours later were used for viral propagation on Vero E6 cells.

For all the viral stock used in flow cytometry analysis experiments, viruses were purified through a 20% sucrose cushion by ultracentrifugation at 80,000xg for 2 hours at 4°C. Pellets were resuspended in HNE1X pH7.4 (Hepes 5 mM, NaCl 150 mM, EDTA 0.1 mM), aliquoted and stored at -80°C. Viral stock titers were determined on Vero E6 cell by plaque assay and are expressed as PFU per ml. Virus stocks were also determined by flow cytometry as previously described ^{38,39} Briefly, Vero E6 cells were incubated for 1h with 100µl of 10-fold serial dilutions of viral stocks. The inoculum was then replaced with 500µl of culture medium and the percent of E2 expressing cells was quantified by flow cytometry at 8 hpi. Virus titers were calculated using the following formula and expressed as FACS Infectious Units (FIU) per ml. [Titer (FIU/ml) = (average % of infection) x (number of cells in well) x (dilution factor) / (ml of inoculum added to cells)].

**Reagents..** The following antibodies were used: anti-FHL1 mAb (ref MAB5938, R & D Systems), anti-FHL1 rabbit Ab (ref NBP1-88745, Novus Biologicals), anti-vimentin antibody (ab24525, abcam), anti-GAPDH mAb (ref SC-47724, Santa Cruz Biotechnology), polyclonal rabbit anti-HA (ref 3724, Cell Signaling Technology), anti-FLAG M2 mAb (ref F1804, SIGMA), anti-RFP (ref 6G6, Chromotek), anti-CHIKV E2 mAb (3E4 and 3E4 conjugated-CY3), anti-alphavirus E2 mAb (CHIK-265 was a kind gift from Michael Diamonds, University school of medicine, St Louis, USA), anti-EEEV E1 mAb (ref MAB8754, Sigma), anti-pan-flavivirus E protein mAb (4G2), anti-dsRNA J2 mAb (Scicons), Alexa Fluor^{™} 488-conjugated goat anti-rabbit IgG (A11034, Invitrogen), Alexa Fluor^{™}-647-conjugated goat anti-chicken IgG (ab150175, abcam), Alexa Fluor^{™} 488-conjugated goat anti-mouse IgG (115-545-003, Jackson ImmunoResearch), Alexa Fluor^{™} 647-conjugated goat anti-mouse IgG (115-606-062, Jackson ImmunoResearch), peroxydase-conjugated donkey anti-rabbit IgG (711-035-152, Jackson ImmunoResearch), and anti-mouse/HRP (P0260, Dako Cytomotion). FLAG magnetic beads (ref M8823, SIGMA), HA-magnetic beads (ref 88837, Thermo Fisher Scientific) and anti-RFP coupled to magnetic agarose beads (RFP-Trap MA, Chromotek) were used for immunoprecipitation experiments.

**CRISPR genetic screen.** The GeCKO v2 human CRISPR pooled libraries (A and B) encompassing 123,411 different sgRNA targeting 19,050 genes (cloned in the plentiCRISPR v2) were purchased from GenScript. Lentiviral production was prepared independently for each half-library in 293FT cells by co-transfecting sgRNA plasmids with psPAX2 (Kind gift from Nicolas Manel, Institut Curie, Paris, France) and pCMV-VSV-G at a ratio of 4:3:1 with lipofectamine 3000 (Thermo Fisher Scientific). Supernatants were harvested 48h after transfection, cleared by centrifugation (750 x g for 10 min), filtered using a 0.45 µM filter and purified through a 20% sucrose cushion by ultracentrifugation (80,000 x g for 2 hours at 4°C). Pellets were resuspended in HNE1X pH7.4, aliquoted and stored at -80°C. HAP1 cells were transduced by spinoculation (750 x g for 2 hours at 32°C) with each CRISPR-sgRNA lentiviral libraries at a multiplicity of infection (MOI) of 0.3 and a coverage of 500 times the sgRNA representation. Cells were selected with puromycin for 8 days and expanded. Sixty million cells from each library were pooled and infected with CHIKV21 using a MOI of 1. Simultaneously forty million of non-infected pooled cells were pelleted and kept at -80°C to serve as a reference of the library representation at time of infection. Approximately 5 days after infection, cytopathic effect was detectable and surviving cells were collected 2 weeks later. Genomic DNA was extracted from selected cells or non-infected pooled cells using QIAamp DNA column (Qiagen), and inserted gRNA sequences were amplified and subject to next generation sequencing on an Illumina MiSeq (Plateforme MGX, Institut Génomique Fonctionelle, Montpellier, France). gRNA sequences were analyzed using the MAGeCK software ¹³. Additionaly, gRNA sequences were analyzed using the RIGER software following previously published recommendation⁴⁰.

**FHL1 editing.** FHL1 was validated using two independent sgRNA targeting the exon 3 and exon 4, which are common to all FHL1 isoforms. sgRNAs were cloned into the plasmid lentiCRISPR v2 according to Zhang lab's recommendation. HAP1 and 293FT cells were transiently transfected with the plasmid expressing individual sgRNA and selected with puromycin until all mock-transfected cells died (approximately 72 hours). Transfected cells were used to ascertain gRNA-driven resistance to CHIKV cytopathic effect, and clonal cell lines were isolated by limiting dilution and assessed by immunoblot for FHL1 expression.

**Infection** assay. For infection quantification by flow cytometry analysis, cells were plated in 24-well plates. Cells were infected for 24 (293T) or 48 hours (HAP1) , trypsinized and fixed with 2% (v/v) paraformaldehyde (PFA) diluted in PBS for 15 min at room temperature. Cells were incubated for 30 min at 4°C with 1µg/ml of either the 3E4 anti-E2 mAb for CHIKV strains and ONNV) or the CHIKV 265 anti-E2 mAb for MAYV or the anti-E1 mAb for EEEV or anti-pan-flavivirus E 4G2 for DENV and ZIKV . Ab were diluted in permeabilization flow cytometry buffer (PBS supplemented with 5% FBS, 0.5% (w/v) saponin, 0.1% Sodium azide). After washing, cells were incubated with 1µg/ml of Alexa Fluor 488 or 647-conjugated goat anti-mouse IgG diluted in permeabilization flow cytometry buffer for 30 min at 4°C. Acquisition was performed on an Attune NxT Flow Cytometer (Thermo Fisher Scientific) and analysis was done by using FlowJo software (Tree Star). To assess infectious viral particles release during infection, cells were inoculated for 3 hours with viruses, washed once and then maintained in culture medium over a 72-hour period. At indicated time points supernatants were collected and kept at -80°C. Vero E6 cells were incubated with 10-fold serial dilution of supernatant for 24 hours and E2 expression was quantified by flow cytometry as described above.

For detection of infected cells by immunofluorescence, control and ΔFHL1 HAP1 cells were plated on Lab-Tek II CC2 glass slide 8 wells (Nunc). Cells were inoculated with CHIKV21 strain (MOI of 20) or CHIKV-nsP3-mCherry (MOI of 20) for 48 hours, then washed thrice with cold PBS and fixed with 4% (v/v) PFA diluted in PBS for 20 min at room temperature. CHIKV E2 protein was stained with the 3E4 mAb at 5 µg/ml, followed by a secondary staining with 1µg/ml of Alexa 488-conjugated goat anti-mouse IgG. Both antibodies were diluted in PBS supplemented with 3% (w/v) BSA and 0.1% saponin. Slides were mounted with ProLong Gold antifade reagent containing 4,6-diamidino-2-phenylindole (DAPI) for nuclei staining (Thermo Fisher Scientific).

For colocalization experiments, cells infected with CHIKV-nsP3-mCherry (MOI of 20) were stained with 10 µg/ml of the anti-FHL1 mAb, followed by a secondary staining with 1µg/ml of Alexa 488-conjugated goat anti-mouse IgG.

For detection of dsRNA foci, control and ΔFHL1 293T cells were plated on Lab-Tek II CC2 glass slide 8 wells (Nunc) and infected with CHIKV21 strain (MOI of 50) for 4 or 6 hours. After fixation with 4% (v/v) PFA diluted in PBS, cells were stained with 5 µg/ml of the anti-dsRNA mAb, followed by a secondary staining with 1µg/ml of Alexa 488-conjugated goat anti-mouse IgG. Both antibodies were diluted in PBS supplemented with 3% (w/v) BSA and 0.1% Triton 100X. Of note, no dsRNA foci were detectable at 4hpi.

Fluorescence microscopy images were acquired using a LSM 800 confocal microscope (Zeiss).

**Plasmid constructions.** To generate the C-terminal HA-tagged FHL1 isoforms, the cDNAs of FHL1A (NM_001449.4), FHL1B (XM_006724746.2) and FHL1C (NM_001159703.1) were purchased from Genscript. Coding sequence (CDS) were amplified and cloned into pLVX-IRES-ZsGreen1 vector (Takara). Using the same approach, coding sequence of murine FHL1 (NM_001077362.2) and cloned into pLVX-IRES-ZsGreen1 vector. C-terminal HA-tagged FHL2 coding sequence was synthesized by Genscript and subcloned into pLVX-IRES-ZsGreenl vector. The plasmids pCI-neo-3×FLAG plasmids expressing the CHIKV nsP3 and nsP4, the Sindbis virus (SINV) and Semliki Forest virus (SFV) nsP3 proteins were previously described⁴¹. The CHIKV nsP3 ΔHVD, ΔR1 to ΔR4 were generated by site-directed mutagenesis (QuickChange XL Site-Directed Mutagenesis Kit, Agilent)

The plasmids expressing the chimeric nsP3 CHIKV-HVD SINV and nsP3 SINV-HVD CHIKV were obtained as follows. First, the DNA sequence coding for the N-terminal parts of the CHIKV or SINV nsP3 (MD-AUD region) are obtained by PCR using the pCI-neo-3×FLAG expression plasmids as templates and the following sets of primers: 3xFLAG_NotI-F and Overlap-CHIKV-SINV-R, or 3xFLAG_NotI-F and Overlap-SINV-CHIKV-R for CHIKV and SINV constructs, respectively. HVD coding sequences were also generated by PCR using the following primers: Overlap-CHIKV-SINV-F and nsP3-SINV_BamHI-R for SINV HVD, and Overlap-SINV-CHIKV-F and nsP3-CHIKV_BamHI-R for CHIKV HVD. Next, the CHIKV-HVD-SINV and SINV-HVD-CHIKV PCR-fragments were obtained by overlap extension PCR using the previously obtained PCR-products and the following sets of primers: 3XFLAG_NotI-F and nsP3-SINV_BamHI-R or nsP3-CHIKV_BamHI-R. Finally, the chimeric PCR fragments were cloned into a NotI-BamHI digested pLVX-IRES-ZsGreenl vector (Takara).

The plasmid expressing FHL1A-R4 and FHL1A-R4* fusion proteins were obtained by overlap extension PCR approach as well. First, the FHL1A part which is common to both constructs was amplified from a cDNA template (Genscript, NM_001449.4). Second, nsP3-R4 and -R4* portions were obtained by PCR using either the pCI-neo-3×FLAG-nsP3 expression plasmid or the pCHIKV-SG45-R4* plasmid (containing the randomized R4 region) as templates. Next, the FHL1A-R4 and FHL1A-R4* PCR-fragments were obtained by PCR using the previously obtained PCR-products and the outer sets of primers: FHL1A Fwd and FHL1-fusion-Rev or FHL1-fusion-Rand-Rev. Amplification fragments were cloned into a NotI-EcoRI digested pLVX-IRES-ZsGreenl vector (Takara).

To obtain pCHIKV-M-Gluc a viral sequence encompassing the CHIKV 26S promoter and a part of the capsid protein sequence was amplified from pCHIKV-M, cut with PmeI and BssHII and assembled together with an AgeI-PmeI fragment from pCHIKVRepl-Gluc⁴² into an AgeI-BssHII cut vector. From the resulting plasmid the AgeI-BssHII fragment was released and ligated together with a BssHII-SfiI fragment from pCHIKV-M⁴³ into pCHIKV-M cut with AgeI and SfiI.

To establish pCHKV-Rluc-GAA two PCR fragments were amplified from pCHIKV-WT using primers CHIKV 5590 F and Bo422 or Bo421 and CHIKV 8512 R, respectively. The obtained fragments were fused via PCR amplification using the outer primers CHIKV 5590 F and CHIKV 8512 R. The resulting fragment was cut with AgeI and BglI and inserted into pCHIKV-Rluc cut with the same restriction enzymes.

For generation of CHIKV-Rluc-ΔR4 and CHIKV-Rluc-R4* first PCR fragments encompassing the desired changes were amplified and assembled as follows: 1) CHIKV-Rluc-ΔR4: two fragments amplified from CHIKV-Rluc using Bo408 and Bo1259 or Bo1258 and Bo409, respectively, were fused together using the outer primers Bo408 and Bo409. 2) CHIKV-Rluc-R4*: the randomized sequence cassette was obtained sequentially from three successive PCRs: First PCR fragment was generated using primers Bo1260 and. Then, it was fused at the 5' end with a PCR fragment amplified from CHIKV-Rluc with Bo408 and Bo1262. Next, the resulting fragment is further fused at the 3' end with a PCR fragment amplified from CHIKV-Rluc with Bo1263 and Bo409, using the outer primers Bo408 and Bo409. Finally, the PCR fragments containing the ΔR4 and R4* mutations were cut with SacII and AgeI and fused in each case with a NgoMIV-SacII fragment derived from CHIKV-Rluc (SG45) and were cloned into a NgoMIV-AgeI digested SG45 plasmid.

**Trans-complementation and over expression experiments.** The lentiviral plasmids containing FHL1 isoforms were packaged as described above (see 'CRISPR genetic screen' section). Cells of interest were stably transduced by spinoculation (750 x g for 2 hours at 32°C) with these lentiviruses and, when necessary, sorted for GFP-positive cells by flow cytometry. For trans-complementation assays cells were inoculated with CHIKV21 for 48 hours. Cells were then collected and processed for E2 expression by flow cytometry. For ectopic expression, cells were plated on 24-well plates (5×10⁴) and incubated with CHIKV-M-GLuc and CHIKV21, and either processed for E2 expression by flow cytometry or infectious virus yield quantification on Vero E6 cells.

**Kinetic of infection by qPCR assay.** Control and ΔFHL1 HAP1 cells were plated on 60 mm dishes (400,000 cells) and inoculated with CHIKV21 (MOI of 5). At indicated time point cells were washed thrice with PBS, incubated with trypsin 0.25% for 5 min at 37°C to remove cells surface bound particles, and total RNA was extracted using the RNeasy plus mini kit (Qiagen) according to manufacturer's instruction. cDNAs were generated from 500 ng total RNA by using the Maxima First Strand Synthesis Kit following manufacturer's instruction (Thermo Fisher Scientific). Amplification products were incubated with 1 Unit of RNAse H for 20 min at 37 °C, followed by 10 min at 72°C for enzyme inactivation, and diluted 10-fold in DNAse/RNAse free water. Real time quantitative PCR was performed using a Power Syber green PCR master Mix (Fisher Thermo Scientific) on a Light Cycler 480 (Roche). The primers used for qPCR were: E1-C21_F, E1-C21_R for viral RNA quantification, and Quantitect primers for GAPDH were purchased from Qiagen. The relative expression quantification was performed based on the comparative threshold cycle (C_{T}) method, using GAPDH as endogenous reference control. CHIKV negative strand RNA was quantified as previously described⁴⁴ . Briefly, cDNA were generated from 1µg total RNA using a primer containing a 5' tag sequence CHIKV(-)Tag and the SuperScript II reverse transcriptase following the manufacturer's instruction (Thermo Fisher Scientific). Amplifications products were diluted 10-fold and used for real time quantitative PCR with the following primers CHIKV(-)fwd and CHIKV(-)rev. The 133 bp sequence corresponding to the amplified cDNA was synthesized by Genescript and serially diluted (650 to 6.5×10⁹ genes copies/µl) to generate standard curves.

**Genomic viral RNA transfection and kinetic of viral amplification.** To assess CHIKV RNA replication within the cells, we transfected control and ΔFHL1 cells with capped genomic viral RNA generated from pCHIKV-M-Gluc (see 'Virus strains and culture' section). Cells were plated on 48 well plate (3×10⁴ cells) and transfected with 100 ng of purified RNA using the Lipofectamine MessengerMax reagent according to the manufacturer's instruction (Thermo Fisher Science), and cells were cultured in absence or presence of 15 mM NH₄Cl to prevent subsequent viral propagation. At specific times, cells were washed once with PBS and lyzed with Gaussia lysis buffer. Lysates were kept at -20°C until all samples were collected. Luciferase activity was measured by using the Pierce Gaussia Luciferase Glow assay kit on a TriStar2 LB 942 with 20 µl of cell lysate, 20 µl of substrate and 2s integration time.

The same experimental approach was used to monitor luciferase activity from capped genomic viral RNA generated from pCHIKV-Rluc WT (SG45), pCHIKV-Rluc-GAA, pCHIKV-Rluc-ΔR4 and pCHIKV-Rluc-R4* mutants. Luciferase activity was measured using the Renilla Luciferase assay system (Promega) on a TriStar2 LB 942 with 20 µl of cell lysate, 20 µl of substrate and 2.5s integration time.

**Immunoblot.** Cell pellet were lysed in Pierce^{™} IP Lysis Buffer (Thermo Fisher Scientific) containing Halt^{™} Protease and Phosphatase Inhibitor Cocktail (Thermo Fischer Scientific) for 30 min at 4°C. Equal amount of protein, determined by DC^{™}Protein Assay (BioRad), were prepared in LDS Sample Buffer 4X (Pierce^{™}) containing 25 mM dithiothreitol (DTT) and heated at 95°C for 5 min. Samples were separated on Bolt^{™} 4-12% Bis-Tris gels in Bolt^{®} MOPS SDS Running Buffer (Thermo Scientific), and proteins were transferred onto a PVDF membrane (BioRad) using the Power Blotter system (Thermo Fischer Scientific). Membranes were blocked with PBS containing 0.1% Tween-20 and 5% non-fat dry milk and incubated overnight at 4°C with primary antibody. Staining was revealed with corresponding horseradish peroxidase (HRP)-coupled secondary antibodies and developed using SuperSignal^{™} West Dura Extended Duration Substrate (Thermo Fisher Scientific) following manufacturer's instructions. The signals were acquired through Fusion Fx camera (VILBERT Lourmat).

**Co-immunoprecipitation assay.** HEK-293T cells were plated in 10 cm dishes (5.10⁶ cells/ dish). Twenty-four hours later, the cells were transfected with a total of 15 µg of DNA expression plasmids (7.5 µg of each plasmid in co-transfection assays). Twenty-four hours post-transfection the cells washed once with PBS and collected with a cell scrapper. After 5 min centrifugation (400 x g for 5 min), cells pellets were lysed for 30 min in cold IP lysis buffer supplemented with Halt^{™} Protease and Phosphatase Inhibitor Cocktail, and then cleared by centrifugation for 15 min at 6,000 x g. Supernatants were incubated overnight at 4°C, with either anti-FLAG magnetic beads or HA magnetic beads (see 'reagent' section above). Beads were washed three times with BO15 buffer (20 mM Tris-HCl pH 7.4, 150 mM NaCl, 5 mM MgCl2, 10% Glycerol, 0.5 mM EDTA, 0.05% Triton, 0.1% Tween-20). The retained complexes were eluted twice with either 3xFLAG-peptide (200 µg/ml; SIGMA F4799-4MG) or HA peptide (400 µg/ml; Roche# 11666975001) for 30 min at room temperature. Samples were prepared and subjected to immunoblot as described above. For input, 1% of whole cell lysate were loaded on the gel.

**Bacterial expression, purification and GST pull down assay.** To express nsP3, nsP3ΔHVD as glutathione S-transferase fusion proteins, their respective open reading frame (orf) were subcloned into pGEX-4T-1. Similarly, FHL1A cDNA was subcloned into the pET47b (+) and expressed as a 6xHis fusion protein. The following oligonucleotides were used to amplify nsP3 and nsP3ΔHVD cDNAs (sense: 5'-ccccggaattcATGgcaccgtcgtaccgggtaa-3'; antisense: 5'-ccgctcgagTCAtaactcgtcgtccgtgtctg-3') and FHL1A (sense: 5'-ccggaattccATGgcggagaagtttgactgcc-3'; antisense: 5'-ccgctcgagTTAcagctttttggcacagtc-3'). E.Coli strain BL21 Star (Invitrogen) was transformed with recombinant expression vectors encoding GST-nsP3, GST-nsP3ΔHVD or 6xHis-FHL1A recombinant proteins. Transformed bacteria were induced with isopropylthio-P-Dgalactoside (IPTG) for 3 hours at 37°C. Cells were collected by centrifugation and the pellets were resuspended in lysis buffer containing lysozyme (1 mg/mL), incubated 30 min at 4°C followed by three subsequent freeze-thawed cycles and sonication. The bacterial lysates were centrifuged at 13,000 r.p.m for 20 min and the supernatants were incubated with glutathione-Sepharose beads for GST-nsP3 and GST-nsP3ΔHVD, or Ni-NTA column (Qiagen) for 6xHis-FHL1A. Column washing and recombinant protein elution were performed according to the manufacturer's instructions. Five µL of eluted GST fusion proteins and 3 µL of Ni-NTA eluted 6xHis-FHL1A were analyzed by SDS-PAGE and proteins were visualized by Coomassie staining. For pull-down assay, GST, GST-nsP3 or GST-nsP3ΔHVD bound beads were incubated with 6xHis-FHL1A for 1 hour at 4°C in presence of 100 µM ZnSO₄. The resin was washed extensively with a buffer containing 500 mM KCL. The beads were then resuspended in Laemmli buffer, resolved on SDS-PAGE and the presence of 6xHis-FHL1A was assessed by western blot using anti-FHL1 antibody.

**Genetic analysis, fibroblasts and myoblasts from Emery-Dreifuss muscular dystrophy patients.** Dermal fibroblasts and myoblasts were taken from 4 patients carrying *FHL1* gene mutations. *FHL1* gene was analyzed as previously reported ⁶ as they had, among other symptoms, features reminiscent of Emery-Dreifuss muscular dystrophy. Patients P1, P2 and P3 were previously reported ⁶ with detailed clinical description (respectively as patient F321-3, F997-8 and F1328-4) while patient P4 was not yet published. Briefly, patient P4 had myopathy with joint contractures, hypertrophic cardiomyopathy, vocal cords palsy, short stature, alopecia, skin abnormalities and facial dysmorphism. In this patient, *FHL1* analysis revealed an insertion of a full-length LINE-1 retrotransposon sequence together with poly A tail of unknown length (i.e.,? thereafter) after 27 bp of the start of exon 4 (c.183_184ins [LINE1;?; 171_183]) that results at mRNA level in altered splicing with retention of 108 bp of the inserted LINE sequence leading to predicted premature termination codon and shorter FHL1A (Extended Data Fig. 7b).

**Ethics statement.** All materials (skin and/or muscle biopsies) from patients and controls included in this study were taken with the informed consent of the donors and with approval of the local ethical boards. All the procedures were followed alongside the usual molecular diagnostic procedure during patient follow-up, and in accordance with the ethical standards of the responsible national committee on human experimentation.

**In vivo studies.** Animals were housed in the Institut Pasteur animal facilities accredited by the French Ministry of Agriculture for performing experiments on live rodents. Work on animals was performed in compliance with French and European regulations on care and protection of laboratory animals (EC Directive 2010/63, French Law 2013-118, February 6th, 2013). All experiments were approved by the Ethics Committee #89 (and registered under the reference APAFIS#6954-2016091410257906 v2). Male mice either deficient for FHL1 (FHL1-null) or not (WT littermates) were obtained by crossing heterozygous females for FHL1⁴⁵ with WT male Black Swiss mice. Nine day-old male littermates, both FHL1-null and WT mice, were injected with CHIKV21 (10⁵ PFU/20µl) by intradermal route and viral load was determined in tissues by day 7 post infection. Virus titers in tissue samples were determined on Vero E6 cells by tissue cytopathic infectious dose 50 (TCID50/g). For histology experiments, muscles were snap frozen in isopentane cooled by liquid nitrogen for cryo-sectioning then processed for histological staining (hematoxylin and eosin) or immunolabelling.

**Transmission electron microscopy.** Cells were scrapped and fixed for 24 h in 1% glutaraldehyde, 4% paraformaldehyde, (Sigma, St-Louis, MO) in 0.1 M phosphate buffer (pH 7.2). Samples were then washed in phosphate-buffered saline (PBS) and post-fixed for 1 h by incubation with 2% osmium tetroxide (Agar Scientific, Stansted, UK). Cells were then fully dehydrated in a graded series of ethanol solutions and propylene oxide. Impregnation step was performed with a mixture of (1: 1) propylene oxide/Epon resin (Sigma) and then left overnight in pure resin. Samples were then embedded in Epon resin (Sigma), which was allowed to polymerize for 48 hours at 60°C. Ultra-thin sections (90 nm) of these blocks were obtained with a Leica EM UC7 ultramicrotome (Wetzlar, Germany). Sections were stained with 2% uranyl acetate (Agar Scientific), 5% lead citrate (Sigma) and observations were made with a transmission electron microscope (JEOL 1011, Tokyo, Japan).

**Cell viability assay.** Cell viability and proliferation were assessed using the CellTiter-Glo 2.0 Assay (Promega) according to the manufacturer's protocol. In brief, cells were plated in 48-well plates (3×10⁴). At specific times, 100 µl of CellTiter-Glo reagent were added to each well. After 10 min incubation, 200 µl from each well were transferred to an opaque 96-well plate (Cellstar, Greiner bio-one) and luminescence was measured on a TriStar2 LB 942 (Berthold) with 0.1 second integration time.

**Statistical analysis.** Graphical representation and statistical analyses were performed using Prism7 software (GraphPad Software). Unless otherwise stated, results are shown as means +/- standard deviation (SD) from at least 2 independent experiments in duplicates. Differences were tested for statistical significance using the unpaired two-tailed t test, One-way or Two-way Anova with multiple comparison post-test.

### Results:

Several host factors implicated in CHIKV infection have been identified, however none of them accounts for CHIKV tropism for joint and muscle tissues⁷⁻¹⁰. To identify key host factors dictating CHIKV cell permissiveness, we performed a genome-wide CRISPR-Cas9 screen in the HAP1 haploid cell line (**data not shown**). HAP1 cells expressing the human GeCKO v2 single guide RNA libraries A and B, which contains each 3 unique sgRNAs targeting 19,050 genes¹¹, were inoculated with CHIKV21, a strain isolated from a patient infected during the 2005-2006 CHIKV outbreak in La Reunion Island¹². Genomic DNA from lentivirus-transduced cells that survived to CHIKV infection was isolated, amplified and the corresponding integrated sgRNA sequenced. Gene enrichment was assessed using the MAGeCK software¹³ (**data not shown**). The top hit of our screen was the gene encoding the Four-and-a-Half LIM protein 1 (FHL1) (**data not shown**), the founding member of the FHL protein family¹⁴. FHL1 is characterized by the presence of four and a half highly conserved LIM domains with two zinc fingers arranged in tandem¹⁴. FHL1 is strongly expressed in skeletal muscles and heart^{4,14}. In human, there are three FHL1 splice variants: FHL1A, FHL1B and FHL1C^{4,15,16}. FHL1A is the most abundantly expressed, primarily detected in striated muscles⁴ and fibroblasts¹⁷. The two other variants FHL1B and C are expressed in muscles, brain and testis^{15,16}. We functionally validated the requirement of FHL1 in CHIKV21 infection by using two distinct gRNAs targeting all three FHL1 isoforms (**data not shown**). We generated HAP1 and 293T knockout *FHL1* clones (ΔFHL1) and confirmed gene editing by sequencing and western blot analysis (**data not shown**). FHL1 knockout did not alter cell proliferation and viability as determined by CellTiter-Glo assay (**data not shown**). CHIKV infection and release of infectious particles was drastically inhibited in ΔFHL1 cells (**data not shown**). Trans-complementation of ΔFHL1 cells with a human cDNA encoding FHL1A, but not FHL1B or C, restored both susceptibility to CHIKV21 infection and virus release (**data not shown**), indicating that FHL1A is a critical factor for CHIKV21 infection. Expression of FHL2, a member of the FHL family predominantly expressed in heart¹⁸, restored CHIKV infection in ΔFHL1 cells, albeit to a lower efficiency than FHL1 (**data not shown**). We then assessed FHL1 dependency of CHIKV strains from distinct genotypes. FHL1 is important for infection by strains belonging to the Asian (strain St Martin H20235 2013), the ECSA (East, Central, and South African) strains Ross and Brazza (MRS1 2011) and the Indian Ocean (IOL) (strain M-899) lineages (**data not shown**). Of note, the requirement for FHL1 was less pronounced with CHIKV 37997, a strain from the West African genotype (**data not shown**). We next tested the requirement of FHL1 for infection by other alphaviruses. Interestingly, O'nyong-nyong virus (ONNV), an Old World alphavirus that is phylogenetically very close to CHIKV¹, showed a dramatically reduced infection level in ΔFHL1 cells (**data not shown**). In sharp contrast, other Old World alphaviruses such as Mayaro virus (MAYV), Sindbis virus (SINV), Semliki Forest Virus (SFV) and Ross River virus (RRV), and New World encephalitic viruses such as Eastern equine encephalitis virus (EEEV), Western equine encephalitis virus (WEEV) or Venezuelan equine encephalitis virus (VEEV) infected HAP1 cells in a FHL1-independent manner (**data not shown**)**.** No effect of FHL1 was observed for infection by Dengue virus (DENV) or Zika virus (ZIKV), two members of the *Flavivirus* genus (**data not shown**)**.** Consistent with the requirement of FHL1 for CHIKV infection, BeWo or HepG2 cells which are poorly susceptible to CHIKV infection^{20,21} and do not express endogenous FHL1 (**data not shown**) became permissive to the virus upon FHL1A expression (**data not shown**)**.** This highlights the major role played by FHL1A in human cell permissiveness to CHIKV.

To determine which step in CHIKV life cycle requires FHL1, we challenged parental and ΔFHL1 cells with CHIKV particles and quantified the viral RNA at different time points (**data not shown**)**.** We did not observe any major difference in CHIKV RNA levels in FHL1-deficient cells compared to WT cells at 2h post-infection (**data not shown**)**.** In contrast, a massive reduction of CHIKV RNA was observed in ΔFHL1 cells as early as 6h post-infection (**data not shown**) which was even greater 24h post-infection, suggesting that FHL1 expression is involved in an early post-entry step of the CHIKV life cycle. We therefore bypassed virus entry and uncoating by transfecting CHIKV RNA into controls or ΔFHL1 cells in the presence of NH₄Cl to inhibit further rounds of infection⁹. Upon CHIKV RNA transfection, viral replication was drastically impaired in ΔFHL1 cells compared to WT cells (**data not shown**)**.** To evaluate the contribution of FHL1 in incoming genome translation versus RNA replication, we generated a replication-deficient CHIKV molecular clone (with the GDD motif of the viral polymerase nsP4 mutated to GAA) encoding a *Renilla* luciferase (Rluc) fused to the nsP3 protein as described ²². Transfection of CHIKV GAA RNA in ΔFHL1 or control cells resulted in a similar Rluc activity (**data not shown**), indicating that FHL1 is dispensable for CHIKV incoming RNA translation. When similar experiments were performed with the WT CHIKV RNA, a massive increase in Rluc activity was observed in control cells but not ΔFHL1 24 hpi (**data not shown**), demonstrating that FHL1 is essential for viral RNA replication. Furthermore, qRT-PCR experiments showed that ablation of FHL1 resulted in a severely reduced synthesis of CHIKV negative strand RNA (**data not shown**). We then investigated the impact of FHL1 in the production of dsRNA intermediates which are a marker of viral replication complex (vRC) assembly²³. At 6h post-infection, a massive reduction of dsRNA-containing complexes was observed in ΔFHL1 cells stained with anti-dsRNA mAb when compared to parental cells (**data not shown**). Consistent with this observation, transmission electron microscopy showed that the formation of plasma membrane-associated spherules and cytoplasmic vacuolar membrane structures, which are alphavirus-induced platforms required for viral RNA synthesis²⁴, are absent in ΔFHL1 cells (**data not shown**). Altogether, these data show that FHL1 is critical for CHIKV RNA replication and vRC formation in infected cells.

We next investigated FHL1 location during infection. Confocal microscopy studies showed that FHL1 displays a diffuse cytoplasmic distribution in uninfected human fibroblasts. In cells infected for 6h, FHL1-containing foci appeared and colocalized with nsP3 (**data not shown**), a CHIKV non-structural protein orchestrating viral replication in the cytoplasm^{25,26}. Indeed, CHIKV nsP3 contains a large C-terminal hypervariable domain (HVD)²⁵ known to mediate assembly of protein complexes and regulate RNA amplification^{25,26}. Interestingly, FHL1 and FHL2 have been reported as putative nsP3 HVD binding partners in mass spectrometry analyses ^{26,27}. We experimentally validated FHL1-nsP3 interaction (**data not shown**) and found that endogenous FHL1 co-immunoprecipitates with nsP3 from CHIKV-infected cells (**data not shown**)**.** Consistent with infection studies, both FHL1A and FHL2 coprecipitated with CHIKV nsP3 (**data not shown**)**.** FHL1A-nsP3 interaction is specific for CHIKV as it was not observed with other alphaviruses such as SINV or SFV, which do not depend on FHL1 for infection (**data not shown**)**.** Of note, in ΔFHL1 cells, nsP3 retained its ability to bind G3BP1 and 2, two components of the stress granules implicated in CHIKV replication^{22,26} (**data not shown**). We next generated chimeric proteins where the HVD region of CHIKV nsP3 is swapped with the corresponding domain of SINV nsP3 and vice versa. Whereas CHIKV-SINV(HVD) chimeric protein lost its ability to bind FHL1, the HVD of CHIKV in the context of SINV nsP3 protein conferred binding to FHL1 (**data not shown**). Pull-down experiments with purified proteins showed that FHL1A directly binds to WT nsP3 but not to the HVD-deficient variant (**data not shown**)**.** We then mapped the binding region within CHIKV nsP3HVD responsible for FHL1A interaction (**data not shown**). The FHL1 binding domain, referred as HVD-R4, is found in all CHIKV and ONNV strains and is located upstream of the short repeating peptide corresponding to G3BP1/2 binding sites²⁶ (**data not shown**). Deletion of the HVD-R4 region strongly impaired FHL1 interaction with nsP3, without affecting G3BP1/2 binding to the viral protein (**data not shown**). To investigate whether FHL1 interaction with the HVD region of nsP3 is required for FHL1 proviral role, we generated two chimeric FHL1A protein either fused to the HVD-R4 peptide (FHL1A-R4) or to a randomized peptide sequence of HVD-R4 (FHL1A-R4*) as a positive control (**data not shown**) and assessed their ability to interact with nsP3. Whereas FHL1A-R4 failed to bind nsP3 (**data not shown**), FHL1A-R4* interacted with nsP3 as efficiently as WT FHL1A protein (**data not shown**). These results indicate that the fused HVD-R4 peptide likely hides the binding site of FHL1A to nsP3, inhibiting their interaction. Furthermore, trans-complementation of ΔFHL1 cells with a cDNA encoding FHL1A-R4 did not restore CHIKV21 infection when compared to FHL1A-R4* or WT FHL1A (**data not shown**). Consistent with this, *in vitro* transcribed RNA from CHIKV molecular clone mutated in FHL1 binding site (ΔR4 or R4*) showed a strong defect in replication after transfection in 293T cells (**data not shown**). Together these data strongly suggest that the interaction between the HVD region of nsP3 with FHL1 is critical for FHL1 proviral function.

Mutations in the *FHL1* gene have been associated with X-linked myopathies^{5,28}, including the Emery-Dreifuss muscular dystrophy (EDMD)⁶, a rare genetic disease characterized by early joint contractures, muscular wasting and adult-onset cardiac disease²⁹. We studied the permissiveness to CHIKV of dermal fibroblasts and myoblasts from four EDMD male patients carrying *FHL1* gene mutations as well as from two healthy donors (**data not shown**). A detailed clinical description of P1, P2 and P3 has been reported⁶, and patient P4 presented with EDMD and additional clinical abnormalities (see methods). Analysis of P4 *FHL1* gene revealed the insertion of a full-length LINE-1 retrotransposon sequence in exon 4 (**data not shown**). FHL1 expression is severely reduced in primary cells from all four EDMD patients as established by immunoblot analysis (**data not shown**). Infection studies showed that fibroblasts and myoblasts from those EDMD patients are resistant to CHIKV21 and M-899 Mauritian strains (**data not shown**), and exhibit a massive defect in the release of infectious particles (**data not shown**), in contrast to healthy donor cells. Similar results were obtained with the CHIKV strains Brazza, Ross and H20235 (**data not shown**). FHL1-null myoblasts and fibroblasts remained highly susceptible to MAYV, which does not rely on FHL1 for replication (**data not shown**). Trans-complementation of EDMD fibroblasts by a lentivirus encoding WT FHL1A restored CHIKV viral antigen synthesis (**data not shown**) and infectious particle release (**data not shown**).

To directly assess the role of FHL1 in chikungunya pathogenesis, we conducted *in vivo* experiments in mice expressing or not FHL1. Human and mouse FHL1 orthologues are highly conserved (**data not shown**). Murine FHL1 interacts with CHIKV nsP3 and enhances viral infection, albeit less efficiently that its human orthologue (**data not shown**). Moreover, CHIKV infection was strongly impaired in the murine muscle cell C2C12 deleted for the *fhl1* gene (**data not shown**). Susceptibility to CHIKV infection of young mice deficient or not for FHL1 was then tested. CHIKV actively replicated in tissues of WT littermates, as previously reported²⁰, but virtually no infectious particles were detected in tissues of FHL1-null mice (**Figure 5**). Moreover, necrotizing myositis with massive infiltrates and necrosis of the muscle fibers were observed in skeletal muscle of WT littermates, while FHL-null mouse muscle showed no detectable pathology (**data not shown**). Immunolabelling with Ab against CHIKV E2 protein, FHL1 and vimentin in muscle revealed that in young WT mice, CHIKV mainly targets muscle fiber expressing FHL1, whereas muscle cells of FHL1-null mice show no label for CHIKV nor for FHL1 (**data not shown**). These experiments demonstrate that *FHL1* knock out mice are resistant to CHIKV infection.

In summary, this study shows that FHL1 is a critical CHIKV host dependency factor for infection and pathogenesis. *In vivo,* FHL1 expression pattern, which accounts for the clinical presentation of EDMD, also reflects CHIKV tissue tropism for skeletal muscles and joints. This suggests that the hijacking of FHL1 by CHIKV during infection may, on top of allowing viral replication, lead to cellular dysfunctions contributing to muscular and joint pains that are the hallmark of chikungunya disease^{1,2}. Mechanistically, FHL1 interacts with the HVD domain of nsP3 to enable viral RNA synthesis and viral replication complex formation. The alphavirus nsP3 HVD domain is an intrinsically disordered region that binds distinct sets of cellular proteins^{23,26,30} such as the G3BP1 and G3PB2, two key components and markers of stress granules that are important for the replication of CHIKV and other alphaviruses^{22,26}. G3BP1/2 nsP3 interactions are thought drive a common alphavirus-specific mechanism that is important for assembly of the replication complex and stabilization of viral G RNA^{22,23,26}. FHL1 interacts with a nsP3 HVD region which is located away from G3BP1/2 binding sites. Therefore, FHL1 and G3BP proteins likely play distinct roles during CHIKV replication. In contrast to G3BPs, FHL1 is selectively used by CHIKV, suggesting that it may accomplish a specific and essential function in CHIKV RNA amplification. Upon interaction with FHL1, CHIKV nsP3 HVD may adopt a unique conformation that is critical for the initiation of viral replication. Interestingly, intrinsically disordered domains (IDD) such as the nsP3 HVD have also been shown to induce liquid-liquid phase separations³¹ and negative-stranded RNA viruses use proteins displaying IDDs to form liquid organelles for their replication³². Indeed, in CHIK-infected cells, nsP3 forms intracellular granules reminiscent of these virus-induced inclusions ^{33,34}. FHL1 may regulate the formation and/or the dynamic of such granules to create an optimal environment for efficient CHIKV RNA amplification. FHL1 contains four LIM domains arranged in tandem known to function as a modular protein binding interface regulating diverse cellular pathways³⁵. FHL1 has been shown to scaffold MAPK components (Raf-1/MEK2/ERK2) to the stretch sensor Titin N2B to transmit MAPK signals that regulate muscle compliance and cardiac hypertrophy^{36,37}. One may speculate that, during CHIKV infection, FHL1 may be hijacked from its physiological function in sarcomere extensibility and intracellular signaling to act as scaffolding protein promoting CHIKV RNA amplification.

In conclusion, this study provides major insights into the understanding of CHIKV interactions with its target host cell. Although other host-factors have been identified as required for CHIKV infection, none of them fully account for the specific joint and muscular pathology which is the hallmark of CHIKV and gave its name to its associated disease, chikungunya, which means "that which bends up" in Makonde, to describe the posture of patient with muscle and joint pain. The hijacking of FHL1 by nsP3 during CHIKV infection is unique and constitutes a critical clue that paves the way to fully decipher the pathogenesis of chikungunya disease. Targeting FHL1A-nsP3 interactions now stands as an attractive therapeutic approach to combat CHIKV pathogenesis.

### REFERENCES:

1. Burt, F. J. et al. Chikungunya virus: an update on the biology and pathogenesis of this emerging pathogen. Lancet Infect. Dis. 17, e107-e117 (2017).
2. Silva, L. A. & Dermody, T. S. Chikungunya virus: epidemiology, replication, disease mechanisms, and prospective intervention strategies. J. Clin. Invest. 127, 737-749 (2017).
3. Weaver, S. C., Charlier, C., Vasilakis, N. & Lecuit, M. Zika, Chikungunya, and Other Emerging Vector-Borne Viral Diseases. Annu. Rev. Med. 69, 395-408 (2018).
4. Greene, W. K., Baker, E., Rabbitts, T. H. & Kees, U. R. Genomic structure, tissue expression and chromosomal location of the LIM-only gene, SLIM1. Gene 232, 203-207 (1999).
5. Schessl, J., Feldkirchner, S., Kubny, C. & Schoser, B. Reducing body myopathy and other FHL1-related muscular disorders. Semin. Pediatr. Neurol. 18, 257-263 (2011).
6. Gueneau, L. et al. Mutations of the FHL1 gene cause Emery-Dreifuss muscular dystrophy. Am. J. Hum. Genet. 85, 338-353 (2009).
7. Ooi, Y. S., Stiles, K. M., Liu, C. Y., Taylor, G. M. & Kielian, M. Genome-wide RNAi screen identifies novel host proteins required for alphavirus entry. PLoS Pathog. 9, e1003835 (2013).
8. Karlas, A. et al. A human genome-wide loss-of-function screen identifies effective chikungunya antiviral drugs. Nat. Commun. 7, 11320 (2016).
9. Zhang, R. et al. Mxra8 is a receptor for multiple arthritogenic alphaviruses. Nature 557, 570-574 (2018).
10. Tanaka, A. et al. Genome-Wide Screening Uncovers the Significance of N-Sulfation of Heparan Sulfate as a Host Cell Factor for Chikungunya Virus Infection. J. Virol. 91, 1_22 (2017).
11. Shalem, O. et al. Genome-scale CRISPR-Cas9 knockout screening in human cells. Science 343, 84-87 (2014).
12. Schuffenecker, I. et al. Genome microevolution of chikungunya viruses causing the Indian Ocean outbreak. PLoS Med. 3, e263 (2006).
13. Li, W. et al. MAGeCK enables robust identification of essential genes from genome-scale CRISPR/Cas9 knockout screens. Genome Biol. 15, 554 (2014).
14. Shathasivam, T., Kislinger, T. & Gramolini, A. O. Genes, proteins and complexes: the multifaceted nature of FHL family proteins in diverse tissues. J. Cell. Mol. Med. 14, 2702-2720 (2010).
15. Brown, S. et al. Characterization of two isoforms of the skeletal muscle LIM protein 1, SLIM1. Localization of SLIM1 at focal adhesions and the isoform slimmer in the nucleus of myoblasts and cytoplasm of myotubes suggests distinct roles in the cytoskeleton and in nuclear-cytoplasmic communication. J. Biol. Chem. 274, 27083-27091 (1999).
16. Krempler, A., Kollers, S., Fries, R. & Brenig, B. Isolation and characterization of a new FHL1 variant (FHL1C) from porcine skeletal muscle. Cytogenet. Cell Genet. 90, 106-114 (2000).
17. Pen, A. E. et al. A novel single nucleotide splice site mutation in FHL1 confirms an Emery-Dreifuss plus phenotype with pulmonary artery hypoplasia and facial dysmorphology. Eur. J. Med. Genet. 58, 222-229 (2015).
18. Chan, K. K. et al. Molecular cloning and characterization of FHL2, a novel LIM domain protein preferentially expressed in human heart. Gene 210, 345-350 (1998).
19. Rezza, G., Chen, R. & Weaver, S. C. O'nyong-nyong fever: a neglected mosquito-borne viral disease. Pathog. Glob. Health 111, 271-275 (2017).
20. Couderc, T. et al. A mouse model for Chikungunya: young age and inefficient type-I interferon signaling are risk factors for severe disease. PLoS Pathog. 4, e29 (2008).
21. Roberts, G. C. et al. Evaluation of a range of mammalian and mosquito cell lines for use in Chikungunya virus research. Sci. Rep. 7, 14641 (2017).
22. Scholte, F. E. M. et al. Stress granule components G3BP1 and G3BP2 play a proviral role early in Chikungunya virus replication. J. Virol. 89, 4457-4469 (2015).
23. Kim, D. Y. et al. New World and Old World Alphaviruses Have Evolved to Exploit Different Components of Stress Granules, FXR and G3BP Proteins, for Assembly of Viral Replication Complexes. PLoS Pathog. 12, e1005810 (2016).
24. Jose, J., Taylor, A. B. & Kuhn, R. J. Spatial and Temporal Analysis of Alphavirus Replication and Assembly in Mammalian and Mosquito Cells. mBio 8, 1-16 (2017).
25. Götte, B., Liu, L. & McInerney, G. M. The Enigmatic Alphavirus Non-Structural Protein 3 (nsP3) Revealing Its Secrets at Last. Viruses 10, 1-26 (2018).
26. Meshram, C. D. et al. Multiple Host Factors Interact with Hypervariable Domain of Chikungunya Virus nsP3 and Determine Viral Replication in Cell-Specific Mode. J. Virol. (2018). doi:10.1128/JVI.00838-18
27. Mutso, M. et al. Mutation of CD2AP and SH3KBP1 Binding Motif in Alphavirus nsP3 Hypervariable Domain Results in Attenuated Virus. Viruses 10, (2018).
28. Schessl, J. et al. Proteomic identification of FHL1 as the protein mutated in human reducing body myopathy. J. Clin. Invest. 118, 904-912 (2008).
29. Bonne, G., Leturcq, F. & Ben Yaou, R. Emery-Dreifuss Muscular Dystrophy. in GeneReviews® (eds. Adam, M. P. et al.) (University of Washington, Seattle, 1993).
30. Frolov, I., Kim, D. Y., Akhrymuk, M., Mobley, J. A. & Frolova, E. I. Hypervariable Domain of Eastern Equine Encephalitis Virus nsP3 Redundantly Utilizes Multiple Cellular Proteins for Replication Complex Assembly. J. Virol. 91, (2017).
31. Uversky, V. N. Intrinsically disordered proteins in overcrowded milieu: Membrane-less organelles, phase separation, and intrinsic disorder. Curr. Opin. Struct. Biol. 44, 18-30 (2017).
32. Nikolic, J. et al. Negri bodies are viral factories with properties of liquid organelles. Nat. Commun. 8, 58 (2017).
33. Fros, J. J. et al. Chikungunya Virus nsP3 Blocks Stress Granule Assembly by Recruitment of G3BP into Cytoplasmic Foci. J. Virol. 86, 10873-10879 (2012).
34. Remenyi, R. et al. Persistent Replication of a Chikungunya Virus Replicon in Human Cells Is Associated with Presence of Stable Cytoplasmic Granules Containing Nonstructural Protein 3. J. Virol. 92, (2018).
35. Kadrmas, J. L. & Beckerle, M. C. The LIM domain: from the cytoskeleton to the nucleus. Nat. Rev. Mol. Cell Biol. 5, 920-931 (2004).
36. Sheikh, F. et al. An FHL1-containing complex within the cardiomyocyte sarcomere mediates hypertrophic biomechanical stress responses in mice. J. Clin. Invest. 118, 3870-3880 (2008).
37. Raskin, A. et al. A novel mechanism involving four-and-a-half LIM domain protein-1 and extracellular signal-regulated kinase-2 regulates titin phosphorylation and mechanics. J. Biol. Chem. 287, 29273-29284 (2012).
38. Medina, F. et al. Dengue virus: isolation, propagation, quantification, and storage. Curr. Protoc. Microbiol. Chapter 15, Unit 15D.2. (2012).
39. Meertens, L. et al. The TIM and TAM families of phosphatidylserine receptors mediate dengue virus entry. Cell Host Microbe 12, 544-557 (2012).
40. Joung, J. et al. Genome-scale CRISPR-Cas9 knockout and transcriptional activation screening. Nat. Protoc. 12, 828-863 (2017).
41. Pellet, J. et al. ViralORFeome: an integrated database to generate a versatile collection of viral ORFs. Nucleic Acids Res. 38, D371-378 (2010).
42. Gläsker, S. et al. Virus replicon particle based Chikungunya virus neutralization assay using Gaussia luciferase as readout. Virol. J. 10, 235 (2013).
43. Kümmerer, B. M., Grywna, K., Gläsker, S., Wieseler, J. & Drosten, C. Construction of an infectious Chikungunya virus cDNA clone and stable insertion of mCherry reporter genes at two different sites. J. Gen. Virol. 93, 1991-1995 (2012).
44. Plaskon, N. E., Adelman, Z. N. & Myles, K. M. Accurate strand-specific quantification of viral RNA. PloS One 4, e7468 (2009).
45. Domenighetti, A. A. et al. Loss of FHL1 induces an age-dependent skeletal muscle myopathy associated with myofibrillar and intermyofibrillar disorganization in mice. Hum. Mol. Genet. 23, 209-225 (2014).

## Claims

1. A method for identifying a substance useful for inhibiting the replication capacity of chikungunya virus (CHIKV) comprising the steps of (a) contacting a polypeptide (P1) containing an amino acid sequence of the human FHL1 protein with a polypeptide (P2) having an amino acid sequence of the CHIKV NSP3 protein, under conditions and for a time sufficient to permit binding and the formation of a complex between the two polypeptides (P1) and (P2), in the presence of a test substance, and (b) detecting the formation of the complex, in which the ability of the test substance to inhibit the interaction between the two polypeptides (P1) and (P2) is indicated by a decrease in complex formation as compared to the amount of complex formed in the absence of the test substance and (c) selecting the substance that inhibits the interaction.

2. The method of claim 1 wherein the polypeptide (P1) comprises an amino acid sequences having at least 90% of identity with the amino acid sequence as set forth in SEQ ID NO:1, SEQ ID NO:2 or SEQ ID NO:3.

3. The method of claim 1 wherein the polypeptide (P2) comprises an amino acid sequence having at least 90% of identity with the amino acid sequence ranging from the amino acid residue at position R326 to amino acid residue at position L524 in SEQ ID NO:4.

4. The method of claim 1 wherein the polypeptide (P1) and/or (P2) is labelled with a detectable molecule.

5. The method of claim 1 wherein the step (b) consists in generating physical values which illustrate or not the ability of said test substance to inhibit the interaction between the polypeptides (P1) and (P2) and comparing said values with standard physical values obtained in the same assay performed in the absence of the said test substance, and wherein encompass light absorbance values, radioactive signals and intensity value of fluorescence signal.

6. The method of claim 5 wherein if after the comparison of the physical values with the standard physical values, it is determined that the said test substance inhibits the binding between polypeptides (P1) and (P2), then the candidate is positively selected at step (c).

7. The method of claim 1 wherein step (b) involves an assay selected from the group consisting of a two-hybrid assay, a gel migration assay, an assay that includes the use of an optical biosensor, an assay that includes the use of affinity chromatography, and an assay that involves detection of a fluorescence signal.

8. The method of claim 1 which further comprises the step (d) consisting in determining whether the substance selected at step (c) inhibits the replication of CHIKV in a host cell in vitro and a step (e) that consists in positively selecting the test substance capable of inhibiting the replication of said CHIKV in said host cell.

9. The method of claim 8 that comprises the steps consisting of i) infecting said host cell with said CHIKV and ii) culturing said infected cell in presence of the test substance, iii) comparing the replicating capacity of the virus with the replication capacity determined in the absence of the test substance and iv) positively selecting the test substance that provides a decrease in the replication capacity of the virus.

10. A method of testing whether a subject is predisposed a CHIKV infection comprising the steps consisting of i) measuring the expression level of FHL1 in a sample obtained from the subject and ii) comparing the expression level measured at step i) with a predetermined reference value and iii) concluding that the subject is predisposed to a CHIKV infection when differential between the measured expression level and the predetermined reference value is detected.

11. A method of testing a subject thought to have or be predisposed to having a CHIKV infection, which comprises the step of analysing a sample of interest obtained from said subject for detecting the presence of a genetic variant in the gene encoding for FHL1 protein.

12. The method of claim 11 that comprises detecting one or more single nucleotide polymorphisms (SNP).

13. A method of testing a subject thought to have or be predisposed to having a CHIKV infection, which comprises the step of analysing a sample of interest obtained from said subject for detecting post-translational modifications of FHL1 protein.

## Patentansprüche

1. Verfahren zum Identifizieren einer Substanz, die zum Unterdrücken der Replikationsfähigkeit von Chikungunya-Virus (CHIKV) nützlich ist, umfassend die Schritte des (a) Kontaktierens eines Polypeptids (P1), das eine Aminosäuresequenz des menschlichen FHL1-Proteins enthält, mit einem Polypeptid (P2), das eine Aminosäuresequenz des CHIKV-NSP3-Proteins enthält, unter Bedingungen und über eine Zeit, die ausreicht, um eine Bindung und die Bildung eines Komplexes zwischen den zwei Polypeptiden (P1) und (P2) zu ermöglichen, bei Anwesenheit einer Testsubstanz, und (b) Erfassens der Bildung des Komplexes, wobei die Fähigkeit der Testsubstanz, die Interaktion zwischen den zwei Polypeptiden (P1) und (P2) zu unterdrücken, durch eine Verringerung der Komplexbildung verglichen mit der Komplexmenge, die bei Abwesenheit der Testsubstanz gebildet würde, angegeben wird, und (c) Auswählens der Substanz, die die Interaktion unterdrückt.

2. Verfahren nach Anspruch 1, wobei das Polypeptid (P1) eine Aminosäuresequenz umfasst, die mindestens 90% Identität mit der Aminosäuresequenz, wie in SEQ ID NO:1, SEQ ID NO:2 oder SEQ ID NO:3 dargelegt, aufweist.

3. Verfahren nach Anspruch 1, wobei das Polypeptid (P2) eine Aminosäuresequenz umfasst, die mindestens 90% Identität mit der Aminosäuresequenz aufweist, die von dem Aminosäurerest an Position R326 bis Aminosäurerest an Position L524 in SEQ ID NO:4 reicht.

4. Verfahren nach Anspruch 1, wobei das Polypeptid (P1) und/oder (P2) mit einem erfassbaren Molekül markiert ist.

5. Verfahren nach Anspruch 1, wobei der Schritt (b) darin besteht, physikalische Werte zu erzeugen, die die Fähigkeit der Testsubstanz, die Interaktion zwischen den Polypeptiden (P1) und (P2) zu unterdrücken, verdeutlichen oder nicht, und die Werte mit physikalischen Standardwerten zu vergleichen, die in dem gleichen Assay bei Abwesenheit der Testsubstanz durchgeführt erhalten werden, und wobei Lichtabsorbanzwerte, radioaktive Signale und Intensitätswerte von Fluoreszenzsignalen beinhalten.

6. Verfahren nach Anspruch 5, wobei, falls nach dem Vergleich der physikalischen Werte mit den physikalischen Standwerten ermittelt wird, dass die Testsubstanz die Bindung zwischen Polypeptiden (P1) und (P2) unterdrückt, der Kandidat dann positiv im Schritt (c) ausgewählt wird.

7. Verfahren nach Anspruch 1, wobei Schritt (b) einen Assay beinhaltet, der aus der Gruppe ausgewählt wird, die aus einem Zwei-Hybrid-Assay, einem Gelmigrations-Assay, einem Assay, der die Verwendung eines optischen Biosensors einschließt, einem Assay, der die Verwendung von Affinitätschromatographie einschließt, und einem Assay, der die Erfassung eines Fluoreszenzsignals beinhaltet, besteht.

8. Verfahren nach Anspruch 1, das weiter den Schritt (d), der darin besteht, zu ermitteln, ob die in Schritt (c) ausgewählte Substanz die Replikation des CHIKV in einer Wirtszelle in vitro unterdrückt, und einen Schritt (e) umfasst, der darin besteht, die Testsubstanz positiv auszuwählen, die imstande ist, die Replikation des CHIKV in der Wirtszelle zu unterdrücken.

9. Verfahren nach Anspruch 8, das die Schritte umfasst, die bestehen aus i) Infizieren der Wirtszelle mit dem CHIKV und ii) Kultivieren der infizierten Zelle bei Anwesenheit der Testsubstanz, iii) Vergleichen der Replikationsfähigkeit des Virus mit der bei Abwesenheit der Testsubstanz ermittelten Reaktionsfähigkeit und iv) positives Auswählen der Testsubstanz, die eine Verringerung der Replikationsfähigkeit des Virus bereitstellt.

10. Verfahren zum Testen, ob ein Proband für eine CHIKV-Infektion prädisponiert ist, umfassend die Schritte, die bestehen aus i) Messen des Expressionsniveaus von FHIL1 in einer von dem Probanden erhaltenen Probe und ii) Vergleichen des in Schritt i) gemessenen Expressionsniveaus mit einem vorbestimmten Referenzwert und iii) Schlussfolgern, dass der Proband für eine CHIKV-Infektion prädisponiert ist, wenn ein Differenzial zwischen dem gemessenen Expressionsniveau und dem vorbestimmten Referenzwert erfasst wird.

11. Verfahren zum Testen eines Probanden, von dem angenommen wird, dass er prädisponiert ist, eine CHIKV-Infektion zu haben, das den Schritt des Analysierens einer interessierenden Probe, die von dem Probanden erhalten wird, umfasst, um die Anwesenheit einer genetischen Variante im Gen, das für FHL1-Protein codiert, zu erfassen.

12. Verfahren nach Anspruch 11, das ein Erfassen eines oder mehrerer Einzelnukleotid-Polymorphismen (SNP) umfasst.

13. Verfahren zum Testen eines Probanden, von dem angenommen wird, dass er prädisponiert ist, eine CHIKV-Infektion zu haben, das den Schritt des Analysierens einer interessierenden Probe, die von dem Probanden erhalten wird, umfasst, um posttranslationale Modifikationen von FHL1-Protein zu erfassen.

## Revendications

1. Procédé d'identification d'une substance utile pour inhiber la capacité de réplication du virus chikungunya (CHIKV) comprenant les étapes consistant à (a) mettre en contact un polypeptide (P1) contenant une séquence d'acides aminés de la protéine FHL1 humaine avec un polypeptide (P2) présentant une séquence d'acides aminés de la protéine CHIKV NSP3, dans des conditions et pendant une durée suffisantes pour permettre une liaison et la formation d'un complexe entre les deux polypeptides (P1) et (P2), en présence d'une substance de test, et (b) détecter la formation du complexe, la capacité de la substance de test à inhiber l'interaction entre les deux polypeptides (P1) et (P2) étant indiquée par une diminution de la formation du complexe par rapport à la quantité de complexe formée en l'absence de la substance de test et (c) sélectionner la substance qui inhibe l'interaction.

2. Procédé selon la revendication 1, dans lequel le polypeptide (P1) comprend une séquence d'acides aminés présentant au moins 90 % d'identité avec la séquence d'acides aminés telle qu'indiquée dans SEQ ID NO:1, SEQ ID NO:2 ou SEQ ID NO:3.

3. Procédé selon la revendication 1, dans lequel le polypeptide (P2) comprend une séquence d'acides aminés présentant au moins 90 % d'identité avec la séquence d'acides aminés allant du résidu d'acide aminé en position R326 au résidu d'acide aminé en position L524 dans SEQ ID NO:4.

4. Procédé selon la revendication 1, dans lequel le polypeptide (P1) et/ou (P2) est marqué avec une molécule détectable.

5. Procédé selon la revendication 1, dans lequel l'étape (b) consiste à générer des valeurs physiques qui illustrent ou non la capacité de ladite substance de test à inhiber l'interaction entre les polypeptides (P1) et (P2) et à comparer lesdites valeurs avec des valeurs physiques standards obtenues dans le même essai effectué en l'absence de ladite substance de test, et englobant des valeurs d'absorbance de lumière, des signaux radioactifs et une valeur d'intensité de signal de fluorescence.

6. Procédé selon la revendication 5 dans lequel si après la comparaison des valeurs physiques avec les valeurs physiques standards, il est déterminé que ladite substance de test inhibe la liaison entre les polypeptides (P1) et (P2), alors le candidat est sélectionné positivement à l'étape (c).

7. Procédé selon la revendication 1, dans lequel l'étape (b) implique un essai choisi dans le groupe consistant en un essai à deux hybrides, un essai par migration sur gel, un essai qui inclut l'utilisation d'un biocapteur optique, un essai qui inclut l'utilisation d'une chromatographie d'affinité et un essai qui implique une détection d'un signal de fluorescence.

8. Procédé selon la revendication 1 qui comprend en outre l'étape (d) consistant à déterminer si la substance sélectionnée à l'étape (c) inhibe la réplication du CHIKV dans une cellule hôte in vitro et une étape (e) consistant à sélectionner positivement la substance de test capable d'inhiber la réplication dudit CHIKV dans ladite cellule hôte.

9. Procédé selon la revendication 8 qui comprend les étapes consistant à i) infecter ladite cellule hôte avec ledit CHIKV et ii) cultiver ladite cellule infectée en présence de la substance de test, iii) comparer la capacité de réplication du virus avec la capacité de réplication déterminée en l'absence de la substance de test et iv) sélectionner positivement la substance de test qui entraîne une diminution de la capacité de réplication du virus.

10. Procédé pour tester si un sujet est prédisposé à une infection par le CHIKV comprenant les étapes consistant à i) mesurer le niveau d'expression de FHL1 dans un échantillon obtenu auprès du sujet et ii) comparer le niveau d'expression mesuré à l'étape i) avec une valeur de référence prédéterminée et iii) conclure que le sujet est prédisposé à une infection par le CHIKV lorsqu'un différentiel entre le niveau d'expression mesuré et la valeur de référence prédéterminée est détecté.

11. Procédé de test d'un sujet supposé présenter ou être prédisposé à présenter une infection par le CHIKV, qui comprend l'étape d'analyse d'un échantillon d'intérêt obtenu auprès dudit sujet pour détecter la présence d'un variant génétique dans le gène codant pour la protéine FHL1.

12. Procédé selon la revendication 11, qui comprend la détection d'un ou plusieurs polymorphismes nucléotidiques simples (SNP).

13. Procédé de test d'un sujet supposé présenter ou être prédisposé à présenter une infection par le CHIKV, qui comprend l'étape d'analyse d'un échantillon d'intérêt obtenu auprès dudit sujet pour détecter des modifications post-traductionnelles de la protéine FHL1.
